(19) Europäisches Patentamt
European Patent Office
Office européen des brevets

(11) **EP 3 712 898 A1**

(12) **EUROPEAN PATENT APPLICATION**

(43) Date of publication:
**23.09.2020 Bulletin 2020/39**

(51) Int Cl.:
**G16H 20/40** (2018.01)          **G16H 50/20** (2018.01)
**G16H 50/30** (2018.01)

(21) Application number: **19305348.5**

(22) Date of filing: **21.03.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **INSERM (Institut National de la Santé et de la
Recherche Médicale)**
**75013 Paris (FR)**
• **Université Paris Descartes**
**75006 Paris (FR)**

• **Assistance Publique-Hôpitaux de Paris (APHP)**
**75004 Paris (FR)**

(72) Inventors:
• **LOUPY, Alexandre**
**92200 Neuilly Sur Seine (FR)**
• **AUBERT, Olivier**
**75009 Paris (FR)**
• **JOUVEN, Xavier**
**92140 Clamart (FR)**
• **LEFAUCHEUR, Carmen**
**75011 Paris (FR)**

(74) Representative: **Lavoix**
**2, place d'Estienne d'Orves**
**75441 Paris Cedex 09 (FR)**

(54) **METHOD OF PREDICTING WHETHER A KIDNEY TRANSPLANT RECIPIENT IS AT RISK OF HAVING ALLOGRAFT LOSS**

(57)     Organ transplantation is currently recognised as the treatment of choice for patients with end-stage renal disease (ESRD), which is an underestimated but increasing burden worldwide. Despite the pressing need for improving patients risk stratification raised by transplant societies as well as regulatory agencies, no risk-stratification system exists that adequately predicts transplant patients' individual risk of allograft loss. This currently represents a limitation for improving patient management, as well as for defining early surrogate end points for clinical trials and development of pharmaceutical agents. The inventors now report the development and validation of an integrative risk prediction score to predict kidney allograft survival of individual patients (NCT03474003). The iBox risk prediction score is the first integrative system validated in several independent populations from Europe & North America as well as across 3 clinical trials (NCT01079143,          EudraCT2007-003213-13, NCT01873157) covering distinct clinical scenarios. In particular, the advantages brought by the iBox risk prediction score are i) improved discrimination performance by combining traditional prognostic factors with mechanistically informed parameters, ii) outperformance when compared with currently existing scoring systems, iii) generalisability when assessed in geographically distinct cohorts from Europe and North America, iv) transportability at different times of evaluation post-transplant, v) performance in a variety of clinical scenarios including clinical trials and vi) readily accessible to clinicians and patients by an online tool for patient risk calculation. Thus, the present invention relates to a method of predicting whether a kidney transplant recipient is at risk of having allograft loss by implementing the iBox risk prediction score.

EP 3 712 898 A1

**Description**

**FIELD OF THE INVENTION:**

The present invention relates to a method of predicting whether a kidney transplant recipient is at risk of having allograft loss.

**BACKGROUND OF THE INVENTION:**

End-stage renal disease is estimated to affect 7.4 million persons worldwide.(1, 2) According to data from the World Health Organisation, more than 1,500,000 live with transplanted kidneys, and 80,000 new kidneys are transplanted each year.(3) Despite the considerable advances in short-term outcomes, kidney transplant recipients continue to suffer from late allograft failure, and little improvement has been made over the past 15 years.(4, 5) While the failure of a kidney allograft represents nowadays an important cause of end stage renal disease, it contrasts with the absence of available robust and widely validated prognostication systems for the risk of allograft failure in individual patients.(6) Accurately predicting which patients are at a high risk of allograft loss would help to stratify patients into clinically meaningful risk groups, which may help guide patient monitoring. Moreover, regulatory agencies and medical societies have highlighted the need for an early and robust surrogate endpoint in transplantation that adequately predicts long-term allograft failure.(7) An enhanced ability to predict allograft outcomes would not only inform daily clinical care, patient counselling and therapeutic decisions but also facilitate the performance of clinical trials, which generally lack statistical power because of the low event rates during the first year after transplantation.(8)

Taken individually, parameters such as estimated glomerular filtration rate (eGFR),(9, 10) proteinuria,(11) histology,(12) or human leukocyte antigen (HLA) antibody profiles,(13) fail to provide sufficient predictive accuracy. Previous efforts at developing prognostic systems in nephrology based on various combinations of parameters have been hampered by small sample sizes, the absence of proper validation, limited phenotypic details from registries, the absence of systematic immune response monitoring, and the failure to include key prognostic factors that affect allograft outcome (e.g., donor-derived factors, polyoma virus-associated nephropathy, disease recurrence).(14-16) Finally, no scoring system has been evaluated in large cohorts from different countries with different transplant practices, allocation systems and practice patterns, thereby limiting their exportability, which is an important consideration for health authorities to accept a scoring system as a surrogate endpoint.(17)

**SUMMARY OF THE INVENTION:**

As defined by the claims, the present invention relates to a method of predicting whether a kidney transplant recipient is at risk of having allograft loss.

**DETAILED DESCRIPTION OF THE INVENTION:**

Organ transplantation is currently recognised as the treatment of choice for patients with end-stage renal disease (ESRD), which is an underestimated but increasing burden worldwide. Indeed, chronic kidney disease (CKD) affects 850 million individuals worldwide (in comparison, diabetes, cancer, and HIV/AIDS affect 422, 42, and 37 million individuals worldwide, respectively). Despite the progress made in immunosuppressive regimens, thousands of allografts are failing every year, with immediate consequences for the patients in terms of mortality, morbidity and cost for the society. Recently, it has been shown that the loss of a kidney allograft represents nowadays an important cause of ESRD. Therefore, the possibility to identify accurately patients who are the most likely to lose their renal allograft carries major clinical implications.

Despite the pressing need for improving patients risk stratification raised by transplant societies (e.g., the European Society of Organ Transplantation, the American Society for Transplantation and the American Society of Transplant Surgeons), regulatory agencies (e.g., the European Medicine Agency and the U.S. Food & Drug Administration), no risk-stratification system exists that adequately predicts transplant patients' individual risk of allograft loss. This currently represents a limitation for improving patient management, as well as for defining early surrogate end points for clinical trials and development of pharmaceutical agents.

The inventors now report the development and validation of an integrative risk prediction score to predict kidney allograft survival of individual patients (NCT03474003). The iBox risk prediction score is the first integrative system validated in several independent populations from Europe & North America as well as across 3 clinical trials (NCT01079143, EudraCT2007-003213-13, NCT01873157) covering distinct clinical scenarios. In particular, the advantages brought by the iBox risk prediction score are i) improved discrimination performance by combining traditional prognostic factors with mechanistically informed parameters, ii) outperformance when compared with currently existing scoring systems, iii) generalisability when assessed in geographically distinct cohorts from Europe and North America, iv) transportability at different times of evaluation post-transplant, v) performance in a variety of clinical scenarios including clinical trials and vi) readily accessible to clinicians and patients by an online tool for patient risk calculation.

The first object of the present invention relates to a method of predicting whether a kidney transplant recipient is at risk of having allograft loss comprising the steps of a) assessing for said recipient a plurality of parameters; b) implementing an algorithm on data comprising the parameters assessed at step a) as to obtain an algorithm output; c) determining the probability of allograft loss from the algorithm output of step b) wherein said parameters are i) time of posttransplant risk evaluation; ii) allograft functional parameters that include estimated glomerular filtration rate and proteinuria; iii) allograft histological parameters that include interstitial fibrosis and tubular atrophy, glomerulitis and peritubular capillaritis, interstitial inflammation and tubulitis and transplant glomerulopathy; and iv) recipient immunological profile that includes the presence and level of the immunodominant circulating anti-HLA donor-specific antibodies.

As used herein, the term "recipient" refers to any subject that receives an organ and/or tissue transplant or graft obtained from a donor. The term "donor" as used herein refers to the subject that provides the organ and/or tissue transplant or graft to be transplanted into the recipient. As used herein, the term "kidney transplant recipient" refers to an individual that has undergone kidney transplantation.

As used herein, the term "allograft loss" refers to loss of function in a transplanted organ. In kidney transplant recipients, graft loss often means return to dialysis.

As used herein, the term "risk" in the context of the present invention, relates to the probability that an event will occur over a specific time period, as in the conversion to allograft loss, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (l-p) is the probability of no event) to no conversion. Accordingly, the expression "predicting whether a kidney transplant recipient is at risk of having allograft loss" in the context of the present invention encompasses making a prediction of the probability, odds, or likelihood that allograft loss may occur. The methods of the present invention may be used to make continuous or categorical measurements of the risk of conversion to allograft loss. According to the present invention the prediction is a dynamic prediction. As used herein, the term "dynamic prediction" refers to providing an assessment of probability or likelihood for a subject to develop graft loss, which may change over time. The method of the present invention is particularly suitable to predict the risk of allograft loss at 3, 5, and/or 7 years from the date of prediction.

As used herein, the term "parameter" refers to any characteristic tested when carrying out the method according to the invention. As used herein, the term "parameter value" refers to a value (a number for instance) associated to a parameter.

As used herein, the term "*time from transplant to risk evaluation*" refers to the time that is comprised between the day of transplantation and the day of the risk evaluation, e.g. the day of allograft biopsy. Typically, the time from transplant risk evaluation is comprises between 0 and 120 months.

According to the present invention, allograft functional parameters include estimated glomerular filtration rate and proteinuria.

As used herein, the term "*glomerular filtration rate*" or "GFR" refers to the volume of fluid filtered from the renal (kidney) glomerular capillaries into the Bowman's capsule per unit time. GFR is used to assess renal function in a subject. As used herein, the term "estimated GFR" or "eGFR" refers to an estimate of the Glomerular Filtration Rate or GFR, calculated using the Modification of Diet in Renal Disease (MDRD) equation developed by the Modification of Diet in Renal Disease Study Group described in Levey A S, Bosch J P, Lewis J B, Greene T, Rogers N, Roth D, "A more accurate method to estimate glomerular filtration rate from serum creatinine: a new prediction equation. Modification of Diet in Renal Disease Study Group" Ann. Intern. Med. 130 (6): 461-70 (1999), the contents of which are herein incorporation by reference. Typically, the unit of measurement for eGFR is mL/min/1,73m$^2$. Typically, the eGFR is typically comprises between 0 and 120 mL/min/1,73m$^2$.

As used herein, the term "*proteinuria*" refers to a condition in which excess protein is present in the urine of a subject. In human subjects, proteinuria is often diagnosed by urinalysis. Clinically, proteinuria is expressed as a ratio for urinary protein/creatinine (g/g of creatinine) and said ratio is typically comprises between 0 and 12.

According to the present invention, the allograft histological parameters are assessed according to the Banff Classification that is an international consensus classification for the reporting of biopsies from solid organ transplants. Banff Lesion Scores indeed assess the presence and the degree of histopathological changes in the different compartments of renal transplant biopsies, focusing primarily but not exclusively on the diagnostic features seen in rejection.

In particular, one of the allograft histological parameter is the *interstitial fibrosis/tubular atrophy (IFTA)* that is evaluated by Banff Lesion Score i-IFTA. This score evaluates the extent of inflammation in scarred cortex. The score is assessed as follows:

- i-IFTA0-No inflammation or less than 10% of scarred cortical parenchyma.
- i-IFTA1-Inflammation in 10% to 25% of scarred cortical parenchyma.
- i-IFTA2-Inflammation in 26% to 50% of scarred cortical parenchyma.
- i-IFTA3-Inflammation in >50% of scarred cortical parenchyma.

Another allograft histological parameter is *microcirculation inflammation* that results from the addition of Banff Lesion Score g + Banff Lesion Score ptc. Banff Lesion Score g evaluates the degree of inflammation within glomeruli. Glomerulitis is a form of microvascular inflammation and is a feature of activity and antibody interaction with tissue in antibody-mediated rejection. The score is assessed as follows:

- g0-No glomerulitis.
- g1-Segmental or global glomerulitis in less than 25% of glomeruli.
- g2-Segmental or global glomerulitis in 25 to 75% of glomeruli.
- g3-Segmental or global glomerulitis in more than 75% of glomeruli.

Banff Lesion Score ptc evaluates the degree of inflammation within peritubular capillaries (PTCs). Together with glomerulitis, peritubular capillaritis constitutes microvascular inflammation as a feature of active antibody-mediated rejection or chronic active antibody-mediated rejection. The score is assessed as follows:

- ptc0-Maximum number of leukocytes <3.
- ptc1-At least 1 leukocyte cell in ≥10% of cortical PTCs with 3-4 leukocytes in most severely involved PTC.
- ptc2-At least 1 leukocyte in ≥10% of cortical PTC with 5-10 leukocytes in most severely involved PTC.
- ptc3-At least 1 leukocyte in ≥10% of cortical PTC with >10 leukocytes in most severely involved PTC.

Another allograft histological parameter is the *interstitial inflammation and tubulitis* that results from the addition of Banff Lesion Score i + Banff Lesion Score t. Banff Lesion Score i evaluates the degree of inflammation in nonscarred areas of cortex ("interstitial Inflammation"), which is often a marker of acute T cell-mediated rejection. The score is assessed as follows:

- i0-No inflammation or in less than 10% of unscarred cortical parenchyma.
- i1-Inflammation in 10 to 25% of unscarred cortical parenchyma.
- i2-Inflammation in 26 to 50% of unscarred cortical parenchyma.
- i3-Inflammation in more than 50% of unscarred cortical parenchyma.

Banff Lesion Score t evaluates the degree of inflammation within the epithelium of the cortical tubules ("tubulitis"). The presence of mononuclear cells in the basolateral aspect of the renal tubule epithelium is one of the defining lesion of acute T cell-mediated rejection in kidney transplants. The score is assessed as follows:

- t0-No mononuclear cells in tubules or single focus of tubulitis only.
- t1-Foci with 1 to 4 mononuclear cells/tubular cross section (or 10 tubular cells).
- t2-Foci with 5 to 10 mononuclear cells/tubular cross section (or 10 tubular cells).
- t3-Foci with > 10 mononuclear cells/tubular cross section or the presence of ≥2 areas of tubular basement membrane destruction accompanied by i2/i3 inflammation and t2 elsewhere.

Another allograft histological parameter is the *transplant glomerulopathy* (cg) that is evaluated by Banff cg Score. The score is based on the presence and extent of glomerular basement membrane (GBM) double contours or multilamination in the most severely affected glomerulus. The score is assessed as follows:

- cg0-No GBM double contours by light microscopy (LM) or EM.
- cg1a-No GBM double contours by LM but GBM double contours (incomplete or circumferential) in at least 3 glomerular capillaries by EM, with associated endothelial swelling and/or subendothelial electron-lucent widening.
- cg1b-Double contours of the GBM in 1-25% of capillary loops in the most affected nonsclerotic glomerulus by LM; EM confirmation is recommended if EM is available.
- cg2-Double contours affecting 26 to 50% of peripheral capillary loops in the most affected-glomerulus.
- cg3-Double contours affecting more than 50% of peripheral capillary loops in the most affected-glomerulus.

A further parameter is the *recipient immunological profile* that includes the presence and level of the immunodominant circulating anti-HLA donor-specific antibodies. As used herein, the term "anti-HLA DSA" has its general meaning in the art and refers the donor-specific anti-HLA antibodies present in the subject. There are several sensitive tests known by the skilled man to determine the level of de novo donor-specific anti-HLA antibodies. For instance, an example of a test to determine the level of de novo donor-specific anti-HLA antibodies comprises: screening of antibodies to HLA-A, HLA-B, HLA-C, HLA-DP, HLA-DQ and HLA-DR gene products using Luminex® solid-phase assay (one lambda Labscreen assay) on serum samples. Typically, the level if expressed as mean-fluorescence intensity ("MFI") that is comprised between 0 and 10 000.

As used herein, the term "algorithm" is any mathematical equation, algorithmic, analytical or programmed process, or statistical technique that takes one or more continuous parameters and calculates an output value, sometimes referred to as an "index" or "index value." Non-limiting examples of algorithms include sums, ratios, and regression operators, such as coefficients or exponents, biomarker value transformations and normalizations (including, without limitation, those normalization schemes based on clinical parameters, such as gender, age, or ethnicity), rules and guidelines, statistical classification models, and neural networks trained on historical populations. Of particular use in combining parameters are linear and non-linear equations and statistical classification analyses to determine the relationship between levels of said parameters and the risk of allograft loss. Of particular interest are structural and syntactic statistical classification algorithms, and methods of risk index construction, utilizing pattern recognition features, including established techniques such as cross-correlation, Principal Components Analysis (PCA), factor rotation, Logistic Regression (LogReg), Linear Discriminant Analysis (LDA), Eigengene Linear Discriminant Analysis (ELDA), Support Vector Machines (SVM), Random Forest (RF), Recursive Partitioning Tree (RPART), as well as other related decision tree classification techniques, Shrunken Centroids (SC), StepAIC, Kth-Nearest Neighbor, Boosting, Decision Trees, Neural Networks, Bayesian Networks, Support Vector Machines, and Hidden Markov Models, among others. Other techniques may be used in survival and time to event hazard analysis, including Cox, Weibull, Kaplan-Meier and Greenwood models well known to those of skill in the art.

In some embodiments, the method of the present invention comprises the use of a machine learning algorithm. The machine learning algorithm may comprise a supervised learning algorithm. Examples of supervised learning algorithms may include Average One-Dependence Estimators (AODE), Artificial neural network (e.g., Backpropagation), Bayesian statistics (e.g., Naive Bayes classifier, Bayesian network, Bayesian knowledge base), Case-based reasoning, Decision trees, Inductive logic programming, Gaussian process regression, Group method of data handling (GMDH), Learning Automata, Learning Vector Quantization, Minimum message length (decision trees, decision graphs, etc.), Lazy learning, Instance-based learning Nearest Neighbor Algorithm, Analogical modeling, Probably approximately correct learning (PAC) learning, Ripple down rules, a knowledge acquisition methodology, Symbolic machine learning algorithms, Subsymbolic machine learning algorithms, Support vector machines, Random Forests, Ensembles of classifiers, Bootstrap aggregating (bagging), and Boosting. Supervised learning may comprise ordinal classification such as regression analysis and Information fuzzy networks (IFN). Alternatively, supervised learning methods may comprise statistical classification, such as AODE, Linear classifiers (e.g., Fisher's linear discriminant, Logistic regression, Naive Bayes classifier, Perceptron, and Support vector machine), quadratic classifiers, k-nearest neighbor, Boosting, Decision trees (e.g., C4.5, Random forests), Bayesian networks, and Hidden Markov models. The machine learning algorithms may also comprise an unsupervised learning algorithm. Examples of unsupervised learning algorithms may include artificial neural network, Data clustering, Expectation-maximization algorithm, Self-organizing map, Radial basis function network, Vector Quantization, Generative topographic map, Information bottleneck method, and IBSEAD. Unsupervised learning may also comprise association rule learning algorithms such as Apriori algorithm, Eclat algorithm and FP-growth algorithm. Hierarchical clustering, such as Single-linkage clustering and Conceptual clustering, may also be used. Alternatively, unsupervised learning may comprise partitional clustering such as K-means algorithm and Fuzzy clustering. In some instances, the machine learning algorithms comprise a reinforcement learning algorithm Examples of reinforcement learning algorithms include, but are not limited to, temporal difference learning, Q-learning and Learning Automata. Alternatively, the machine learning algorithm may comprise Data Pre-processing.

In some embodiments, the output obtained by the algorithm at step b) is a score. As used herein, the term "score" refers to a piece of information, usually a number that conveys the result of the subject on a test. A risk scoring system separates a patient population into different risk groups; herein the process of risk stratification classifies the patients into very high-risk, high-risk, intermediate-risk and low-risk groups.

In some embodiments, the score corresponds to the score depicted in **EXAMPLE 2.**

In some embodiments, the algorithm is implemented on a computer using well-known computer processors, memory units, storage devices, computer software, and other components. Typically, the computer contains a processor, which controls the overall operation of the computer by executing computer program instructions which define such operation. The computer program instructions may be stored in a storage device (e.g., magnetic disk) and loaded into memory when execution of the computer program instructions is desired. The computer also includes other input/output devices that enable user interaction with the computer (e.g., display, keyboard, mouse, speakers, buttons, etc.). One skilled in the art will recognize that an implementation of an actual computer could contain other components as well.

In some embodiments, the algorithm is implemented using computers operating in a client-server relationship. Typically, in such a system, the client computers are located remotely from the server computer and interact via a network. The client-server relationship may be defined and controlled by computer programs running on the respective client and server computers. In some embodiments, the results may be displayed on the system for display, such as with LEDs or an LCD. Accordingly, in some embodiments, the algorithm can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet. The computing system can include clients and servers. A client and server are generally remote from each other and typically interact through a communication network. The relationship of client and server arises by virtue of computer programs running on the respective computers and having a client-server relationship to each other.

In some embodiments, the algorithm is implemented within a network-based cloud computing system. In such a network-based cloud computing system, a server or another processor that is connected to a network communicates with one or more client computers via a network. A client computer (e.g. a mobile device, such as a phone, tablet, or laptop computer) may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For instance, the physician may register the parameters (i.e. input data) on, which then transmits the data over a long-range communications link, such as a wide area network (WAN) through the Internet to a server with a data analysis module that will implement the algorithm and finally return the output (e.g. score) to the mobile device.

In some embodiments, the output results can be incorporated in a Clinical Decision Support (CDS) system. These output results can be integrated into an Electronic Medical Record (EMR) system.

The method as disclosed herein is useful for identifying patients with a high risk of allograft loss. In particular, the method of the present invention is particularly suitable for selecting a therapeutic regimen or determining if a certain therapeutic regimen is more appropriate for a patient identified as having a high risk of allograft loss. Typically, this regimen treatment consists of triple therapy regimen comprising a corticosteroid plus a calcineurin inhibitor (e.g. Ciclosporin, Tacrolimus) and an anti-proliferative agent (e.g. Azathioprine, Mycophenolic acid) may be used. mTOR inhibitors (e.g. Sirolimus, Everolimus) also may be used. Anti-CD25 antibodies may be used such as basiliximab. Reducing the level and the production of the DSA and/or protecting the allograft may be achieved using any suitable medical means known to those skilled in the art. In some embodiments, such reduction and protection comprise a therapeutic intervention with the subject such as administration of antithymomcy globulin (ATG), administration of B cell depleting antibodies, administration of proteasome inhibitor (bortezomib), intravenous administration of immunoglobulins, plasmapheresis, administration of anti-C5 antibodies (e.g. eculizumab) and splenectomy. Typical B cell depleting antibodies include but are not limited to anti-CD20 monoclonal antibodies [e.g. Rituximab (Roche), Ibritumomab tiuxetan (Bayer Schering), Tositumomab (GlaxoSmithKline), AME-133v (Applied Molecular Evolution), Ocrelizumab (Roche), Ofatumumab (HuMax-CD20, Gemnab), TRU-015 (Trubion) and IMMU-106 (Immunomedics)], an anti-CD22 antibody [e.g. Epratuzumab, Leonard et al., Clinical Cancer Research (Z004) 10: 53Z7-5334], anti-CD79a antibodies, anti-CD27 antibodies, or anti-CD19 antibodies (e.g. U.S. Pat. No. 7,109,304), anti-BAFF-R antibodies (e.g. Belimumab, GlaxoSmithKline), anti-APRIL antibodies (e.g. anti-human APRIL antibody, ProSci inc.), and anti-IL-6 antibodies [e.g. previously described by De Benedetti et al., J Immunol (2001) 166: 4334-4340 and by Suzuki et al., Europ J of Immunol (1992) 22 (8) 1989-1993, fully incorporated herein by reference]. AMR can also require blood exchanges (Ivlg, plasmatic exchanges) to remove antibodies present in the recipient circulating compartment and targeting the graft. Reciprocally, where the recipient is predicted a low risk of allograft loss, the immunosuppressive therapy can be reduced in order to diminish the potential for drug toxicity.

In some embodiments, the method of the present invention be used to identify patients in need of frequent follow-up by a physician or clinician to monitor the therapeutic regimen. In some embodiments, a patient can be monitored using the method as disclosed herein, and if on a first (i.e. initial) testing the patient is identified as having a high risk of allograft rejection, the patient can be administered an appropriate therapeutic regiment, and on a second testing (i.e. follow-up testing), the patient is identified as having low risk of allograft loss, the patient can be administered with a therapeutic regiment at a maintenance dose. Thus, the method of the present invention is particularly suitable for discriminating responder from non-responder. As used herein the term "responder" in the context of the present disclosure refers to a subject that will achieve a response, i.e. the risk of allograft loss does show a reduction. A non-responder subject includes subjects for whom the risk of allograft loss does not show any reduction or improvement after the treatment.

In some embodiments, screening patients for identifying patients having a high risk of allograft rejection using the method as disclosed herein is also useful to identify patients most suitable or amenable to be enrolled in clinical trial for assessing a therapy for management of allograft, which will permit more effective subgroup analyses and follow-up studies. Furthermore, the method as disclosed herein can be suitable for monitoring patients enrolled in a clinical trial to provide a quantitative measure for the therapeutic efficacy of the therapy which is subject to the clinical trial. Accordingly, the output the algorithm (e.g. the score) represent a suitable surrogate marker for use in a clinical trial for assessing the efficiency of a particular therapy.

In some embodiments, the method of the present invention is also suitable for use as a qualification tool for screening new biomarkers for assessing risk of allograft loss. In some embodiments, the predictive and/or accuracy power of a biomarker may be compared with the output (e.g. score) for assessing the risk of allograft risk in a transplant recipient. Any statistic methods including e.g multivariate and univariate approaches may be used for assessing predictability and accuracy of the biomarkers in comparison with the output of the algorithm.

The invention will be further illustrated by the following figures and examples. However, these examples and figures should not be interpreted in any way as limiting the scope of the present invention.

**FIGURES:**

**Figure 1A: Kaplan-Meier curves of allograft survival rates in the development cohort according to the iBox risk prediction score strata***

**Figure 1B: iBox risk prediction score-based probability of individual kidney allograft survival (derivation cohort).** The histogram applies to the distribution of the iBox risk score according to five groups: grey bars, iBox risk score strata 1; green bars, IBox risk score strata 2; blue bars, iBox risk score strata 3; purple bars, iBox risk score strata 4, and red bars, iBox risk score strata 5. The risk curves indicate the 3-year (black), 5-year (blue) and 7-year (red) allograft survival rate predictions.

**Figure 1C: Calibration plots at 3, 5 and 7 years for the iBox risk prediction score in the derivation cohort.** The vertical axis is the observed proportion of grafts surviving at the time of interest. The average predicted probability (predicted survival; x-axis) was plotted against the Kaplan-Meier estimate (observed overall survival; y-axis). The black line represents the observed events; the grey line represents the perfectly calibrated model; and the blue line represents the optimism-corrected iBox model.

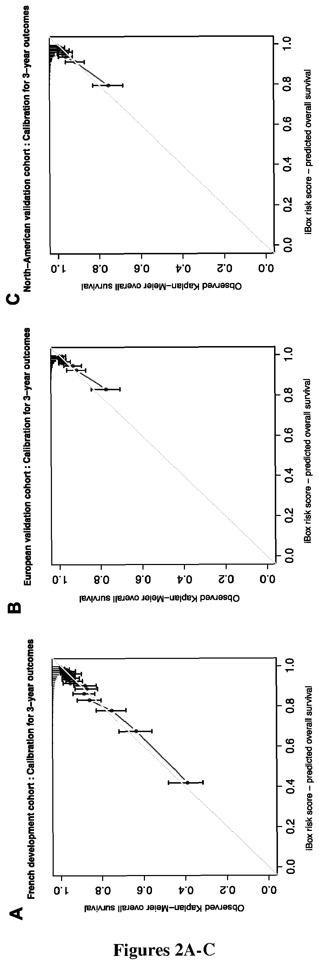

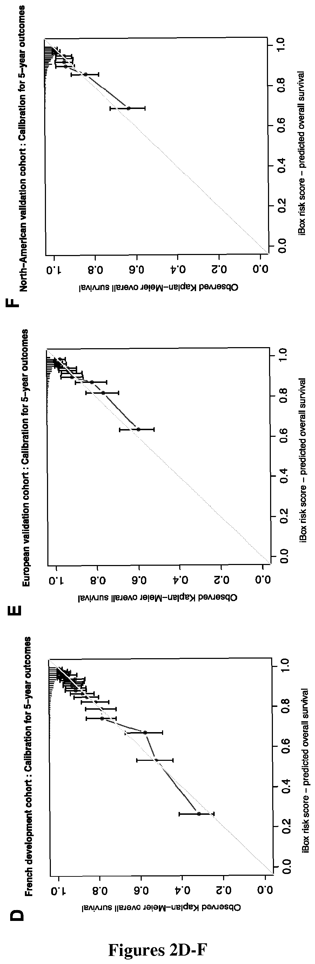

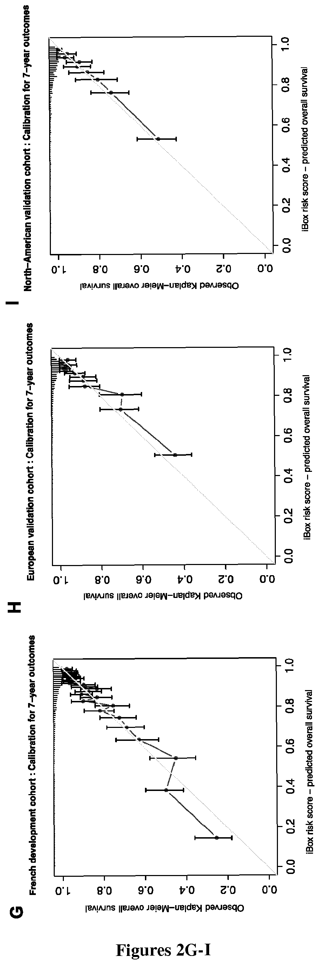

**Figure 2: Calibration plots at 3, 5 and 7 years of the iBox risk scores for the derivation cohort and the validation cohorts.** 3-year (A, B, C), 5-year (D, E, F) and 7-year (G, H, I) predictions. Data are from the development cohort (A, D, G), the European validation cohort (B, E, H) and the North-American cohort (C, F, I). The iBox risk score probabilities were stratified in equally sized subgroups. For each group, the average predicted probability (iBox risk score - predicted survival; x-axis) was plotted against the Kaplan-Meier estimate (observed overall survival; y-axis). The 95% CIs of the Kaplan-Meier estimates are indicated with vertical lines. Dashed line indicates the reference line, indicating where an ideal score would be.

**Figure 3: Density of risk evaluation time points after transplantation**

**Figure 4: Cumulative incidence of graft loss adjusted for competing death for the five iBox risk strata.**

**Figure 5: iBox practical application for clinicians: Ready-to-use interface for clinicians.**

**Figure 6: Distribution and density of the iBox risk scores among the centres.**

**Figure 7: The iBox prediction in Clinical trials and related observed events. Calibration plot at 3, 5 and 7 years.**

**Figure 8: Prognostic nomogram to predict the probability of individual kidney allograft survival using the functional factors eGFR, proteinuria, the time from transplant to iBox risk evaluation (years) and immunodominant circulating anti-HLA DSA MFI.**

**EXAMPLE 1: ALLOGRAFT LOSS RISK PREDICTION SCORE IN KIDNEY TRANSPLANT RECIPIENTS: AN INTERNATIONAL DERIVATION AND VALIDATION STUDY**

**Methods**

*Study design and participants*

*Derivation cohort.* The derivation cohort consisted of 4,000 consecutive patients over 18 years of age who were prospectively enrolled at the time of kidney transplantation from a living or deceased donor at Necker Hospital (n=1,473), Saint-Louis Hospital (n=928), Foch Hospital (n=714), and Toulouse Hospital (n=885) between January 1, 2005, and January 1, 2014, in France. The clinical data were collected from each centre and entered into the Paris Transplant Group database (French data protection authority (CNIL) registration number: 363505). All data were anonymised and prospectively entered at the time of transplantation, at the time of posttransplant allograft biopsies and at each transplant anniversary using a standardised protocol to ensure harmonisation across study centres. Data from the derivation cohort were submitted for an annual audit to ensure data quality (See **EXAMPLE 2** for detailed data collection procedures). Data were retrieved from the database on March 2018. The institutional review boards of the Paris Transplant Group participating centres approved the study. All patients provided written informed consent at the time of transplantation.

*Validation cohorts.* External validation was conducted on 3,557 kidney transplant recipients from a living or a deceased donor over 18 years of age and representing all eligible patients for posttransplant risk evaluation (i.e., undergoing allograft biopsy as part of the standard of care of each centre with adequate biopsy according to the Banff criteria) from six centres: 2,129 recipients recruited in Europe and 1,428 recipients recruited in North America between 2002 and 2014. The European centres included *Hôpital Hôtel Dieu,* Nantes, France (n=632), *Hospices Civils,* Lyon, France (n=608), and the University Hospitals, Leuven, Belgium (n=889). The US centres included the Johns Hopkins Medical Institute, Baltimore, MD (n=580), the Mayo Clinic, Rochester, MN (n=556), and the Virginia Commonwealth University School of Medicine, Richmond, VA (n=292). Data sets from the validation centres were prospectively collected as part of routine clinical practice and entered in the centres' databases in compliance with local and national regulatory requirements and sent anonymised to the Paris Transplant Group.

In France, the transplantation allocation system followed the rules of the French National Agency for Organ Procurement (*Agence de la Biomedecine*). Centres outside of France followed the rules of the Eurotransplant allocation system (Leuven),(18) whereas US centres (Johns Hopkins Hospital, Mayo Clinic and Virginia) followed the rules of the US Organ Procurement and Transplantation System.(19)

*Additional external validation cohort.* Additional external validation was conducted in kidney transplant recipients previously recruited in three registered and published phase II and III clinical trials: a randomised, open-label, multicentre trial that compared a cyclosporine-based immunosuppressive regimen to an everolimus-based regimen in kidney recipients *(Certitem, NCT01079143)*; a randomised, multicentre, double-blind, placebo-controlled trial that investigated the efficacy of rituximab in kidney recipients with acute antibody-mediated rejection *(Rituxerah, EudraCT 2007-003213-13);* and a randomised, double-blind placebo-controlled single-centre trial that investigated the efficacy of bortezomib in kidney recipients with late antibody-mediated rejection *(Borteject, NCT01873157)*.(20-22) The details of the clinical trials depicting the population characteristics, study design, inclusion criteria and interventions are provided in Table 4.

### Candidate predictors

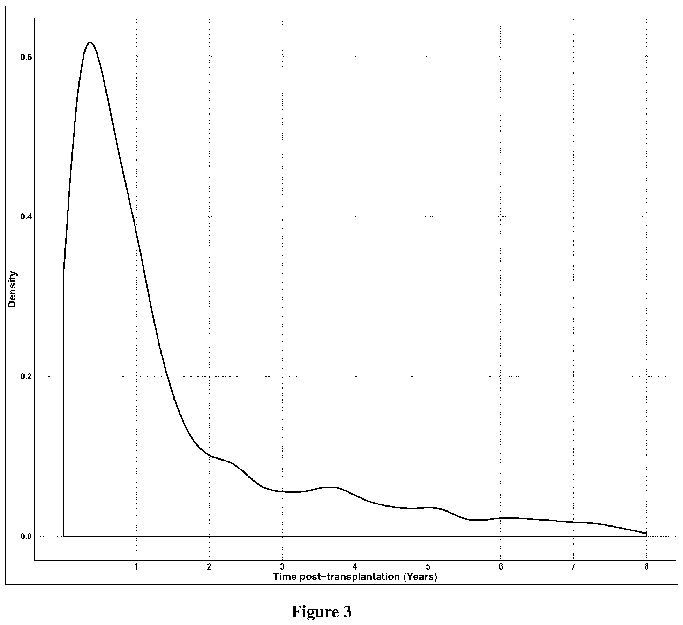

*Posttransplant risk evaluation times.* Risk evaluation after transplantation was conducted at the time of allograft biopsy performed for clinical indication or as *per* protocol, which was performed after transplantation according to the centres' practices. In patients with multiple biopsies, risk evaluation was performed using the date of the first biopsy. The distribution of posttransplant risk evaluation times is provided in **Figure 3.**

Patient risk evaluation after transplant comprised demographic characteristics (including recipient comorbidities, age, gender and transplant characteristics), biological parameters (including kidney allograft function, proteinuria, and circulating anti-HLA antibody specificities and levels), and allograft pathology data (including elementary lesion scores and diagnoses), All these factors are commonly and routinely collected in kidney transplant centres worldwide.

See **EXAMPLE 2** for the list of all prognostic determinants assessed from the derivation cohort.

*Measurements performed at the time of risk evaluation.* Kidney allograft function was assessed by the glomerular filtration rate estimated by the Modification of Diet in Renal Disease Study equation (eGFR) and proteinuria level using the protein/creatinine ratio in the derivation and validation cohorts. Circulating donor-specific antibodies against HLA-A, HLA-B, HLA-Cw, HLA-DR, HLA-DQ and HLA-DP were assessed using single-antigen flow bead assays in the derivation cohort (see **EXAMPLE 2**) and according to local centre practice in the validation cohorts. Kidney allograft pathology data, including elementary lesion scores and diagnoses, were recorded according to the Banff classification in the derivation and validation cohorts (see **EXAMPLE 2**). All the measurements (eGFR, proteinuria, histopathology and circulating anti-HLA DSA) were performed on the day of risk evaluation.

### Outcome

The outcome of interest was allograft loss defined as a patient's definitive return to dialysis or preemptive kidney retransplantation. This outcome was prospectively assessed in the derivation and validation cohorts at each transplant anniversary up to March 31, 2018.

Patient death was considered as a competing event (see **EXAMPLE 2**).

*Missing data*

A total of 59 patients (0.01%) were excluded from the final model due to at least one missing data point.

*Statistical analysis*

We followed the TRIPOD (Transparent Reporting of a Multivariable Prediction Model for Individual Prognosis or Diagnosis) statement for reporting multivariable prediction model development and validation.(23)

Continuous variables are described using means and standard deviations (SDs) or median and the interquartile range. We compared means and proportions between groups using Student's t-test, analysis of variance (ANOVA) (Mann-Whitney test for MFI) or the chi-square test (or Fisher's exact test if appropriate). Graft survival was estimated using the Kaplan-Meier method. The duration of follow-up started with the patient risk evaluation (starting point) up to the date of kidney allograft loss, or at the end of the follow-up (March 31, 2018). For patients who died with a functioning allograft, allograft survival was censored at the time of death as a surviving or functional allograft.(24) A competing risk approach was applied to consider the potential competition of patient death with kidney allograft failure (see **EXAMPLE 2**).(25)

In the derivation cohort, the associations between allograft failure and clinical, histologic, functional, and immunologic factors measured at the patient risk evaluation (see above) were assessed using univariable Cox regression analyses. Hazard proportional assumptions were tested using the log graphic method. The factors identified in these analyses were thereafter included in a final multivariable model.

The internal validity of the final model was confirmed using a bootstrap procedure, which involved generating 1,000 datasets derived from resampling the original dataset and permitting the estimation of the biased corrected 95% CI and the accelerated bootstrap (BCA) HR.(26)

The centre effect was tested in stratified analyses. Potential nonlinear relationships between continuous predictors and graft loss were first investigated using restricted cubic splines and fractional polynomial methods (see **EXAMPLE 2**).

The accuracy of the prediction model was assessed based on its discrimination ability and calibration performance. The discrimination ability (i.e., the ability to separate patients with different prognoses) of the final model was evaluated using Harrell's concordance index (C-index) (see **EXAMPLE 2**).(27) One thousand random samples of the population were used to derive the 95% confidence intervals (CIs) for the C-index. Calibration and goodness of fit (the ability to provide unbiased survival predictions in groups of similar patients) were assessed based on a visual examination of the calibration plots and tested with an extension of the Hosmer-Lemeshow test for survival data. Net reclassification improvement for censored survival data was computed using the SurvIDINRI package in R. (28, 29) The external validity of the final model was thereafter evaluated in the external validation cohorts, including discrimination tests and model calibration as mentioned above.

A risk prediction score ("integrative box risk prediction score, iBox") was calculated for each patient according to the β-regression coefficients estimated from the final multivariable Cox model and normalised to a range between 0 and 5 (see **EXAMPLE 2**). To obtain a reasonable spread of risk, we chose to work on five prognostic risk groups. Cox's method was applied to determine optimal nonarbitrary cut-off points to define five risk groups. (30)

All analyses were performed using R (version 3.2.1, R Foundation for Statistical Computing). Values of $p<0.05$ were considered significant, and all tests were 2-tailed. Details regarding the interpretation of important statistical concepts are given in **EXAMPLE 2.**

## Results

*Characteristics of the derivation and validation cohorts*

The derivation cohort (n=4,000) and the two validation cohorts (n=3,557) comprised a total of 7,557 participants. The characteristics of the derivation and validation cohorts (overall, European and North American validation cohorts) as well as the transplant procedures, policies and allocation systems are detailed in **Table 1 and Tables 5, 6, and 7.** The distribution of the time of posttransplant risk evaluation is provided in **Figure 3**. The median time from kidney transplantation to posttransplant patient risk evaluation was 0.98 years (IQR: 0.27 to 1.07) in the derivation cohort and 0.99 years (IQR: 0.18 to 1.04) in the validation cohort. After a median follow-up after transplantation of 7.65 years (IQR: 5.20 to 10.30) in the derivation cohort and 6.69 years (IQR: 5.39 to 8.21) in the validation cohorts, 1,067 patients (14.12%) developed the primary outcome of allograft loss, 626 (15.65%) in the derivation cohort and 441 (12.40%) in the external validation cohort.

*Prediction of kidney allograft failure in the derivation cohort*

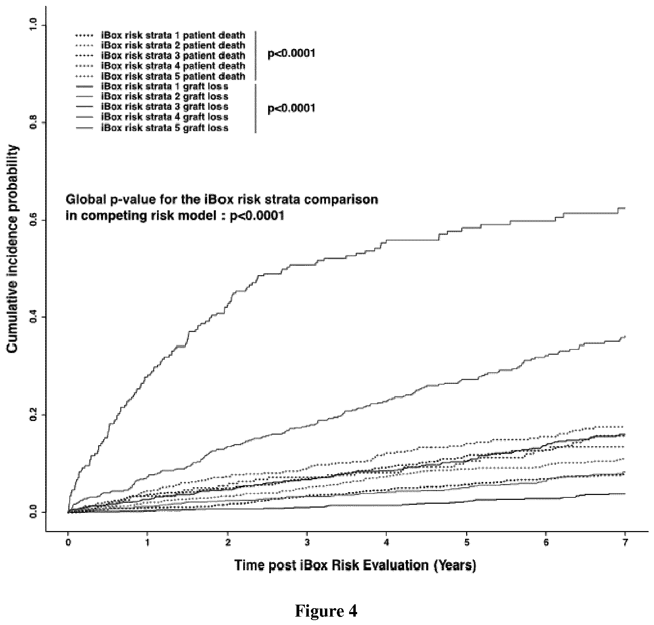

We first investigated the prognostic factors measured at the time of posttransplant risk evaluation that were associated with long-term kidney allograft failure in a univariable analysis. These factors included recipient demographics, transplant characteristics, allograft functional parameters, immunological parameters, and allograft histopathology (**Table 2A**). In the multivariable analysis, the following independent predictors of long-term allograft failure were identified: i) *time of posttransplant risk evaluation* (p=0.005); ii) allograft functional parameters, including *estimated glomerular filtration rate* (eGFR; p<0.001) and *proteinuria* (logarithmic transformation, p<0.001); iii) allograft histological parameters, including *interstitial fibrosis and tubular atrophy* (p=0.031), *microcirculation inflammation* defined by glomerulitis and peritubular capillaritis (p=0.001), *interstitial inflammation and tubulitis* (p=0.014) and *transplant glomerulopathy* (p=0.004); and iv) recipient immunological profile as defined by the *presence and level of the immunodominant circulating anti-HLA donor-specific antibodies* (p<0.001; **Table 2B**). To test the centre effect, we stratified the final multivariable model by transplant centres and confirmed that the eight prognostic parameters identified in the primary analysis remained independently associated with allograft survival (**Table 8**). Using competing risk regression models, we confirmed that the allograft survival analyses performed in the final model were not affected by competition with patient death (see **EXAMPLE 2**, and **Figure 4** for death competing risk analyses).

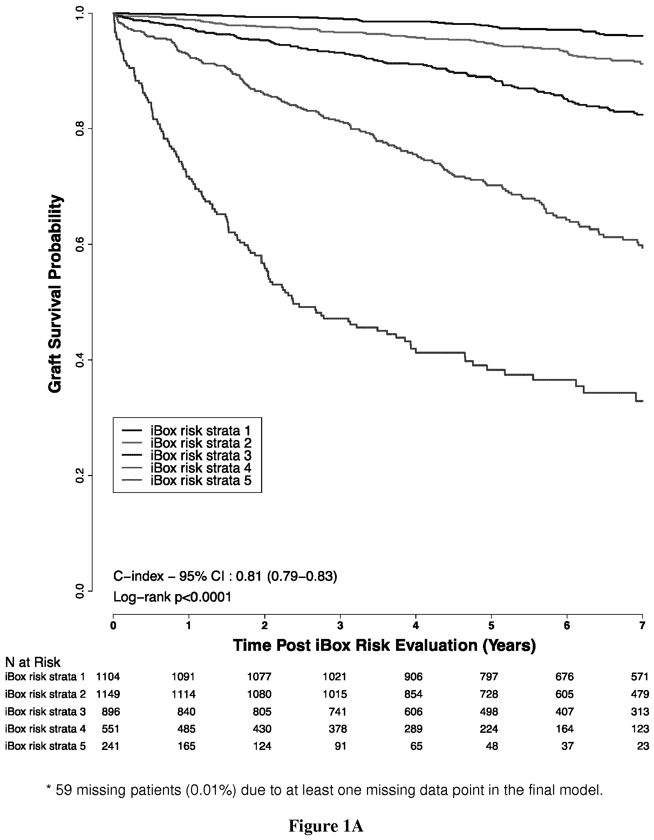

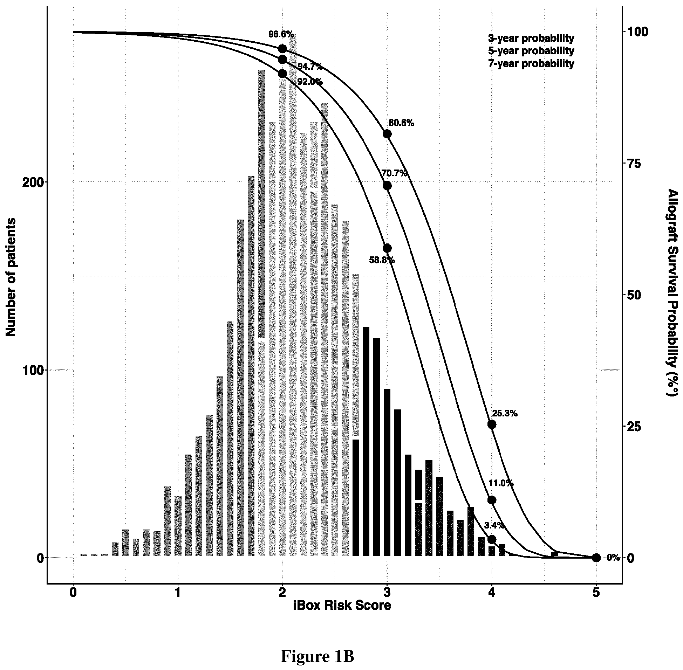

The prognostic score, named iBox, was calculated for each patient according to the β-regression coefficients estimated from the final multivariable Cox model and normalised to a range between 0 and 5 (see **EXAMPLE 2**). The population was divided into five risk groups with an increasingly higher risk of graft loss corresponding to the following cut-off points: iBox risk strata 1 (n=1,104): <1.805; iBox risk strata 2 (n=1,149): 1.805-2.265; iBox risk strata 3 (n=896): 2.265-2.705; iBox risk strata 4 (n=551): 2.705-3.275; and iBox risk strata 5 (n=241): ≥3.275. This stratification achieved a clear separation of the Kaplan-Meier curves, defining five subgroups of patients with distinct long-term allograft prognoses, with 7-year post-risk evaluation allograft survival rates of 96% (95% CI: 94 to 97), 91% (95% CI: 89 to 93), 82% (95% CI: 79 to 85), 59% (95% CI: 54 to 65), and 33% (95% CI: 26 to 41) in strata 1, 2, 3, 4 and 5, respectively (**Figure 1A & 1B**). Based on this score, we built an online ready-to-use interface for the clinician to provide allograft survival estimates for individual patients (http://www.paristransplantgroup.org). We are also providing in **EXAMPLE 4** examples of clinical use of iBox risk prediction scoring in daily practice.

*Prediction model performance in the internal and external validation cohorts*

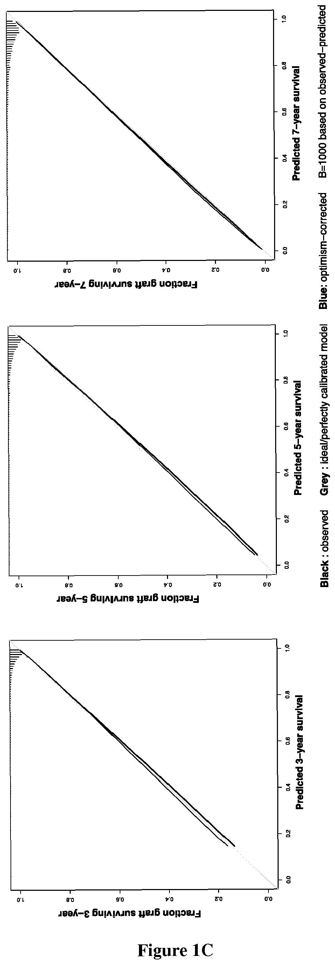

We first internally validated the final multivariable model *via* a bootstrapping procedure with 1,000 samples from the original dataset of the derivation cohort (**EXAMPLE 2**). Using this approach, we confirmed 1) the robustness of the final multivariable model (bias-corrected HRs and 95% CIs, **Table 2B**); 2) the successful discrimination ability at 3, 5 and 7 years (C-index: 0.83, 95% bootstrap percentile CI=0.81 to 0.86; 0.82, 95% bootstrap percentile CI=0.80 to 0.84; 0.81; 95% bootstrap percentile CI=0.79 to 0.83, respectively) of the model; and 3) the accurate calibration at 3, 5 and 7 years (p=0.85, p=0.65 and p=0.36, respectively) (**Figure 1C**).

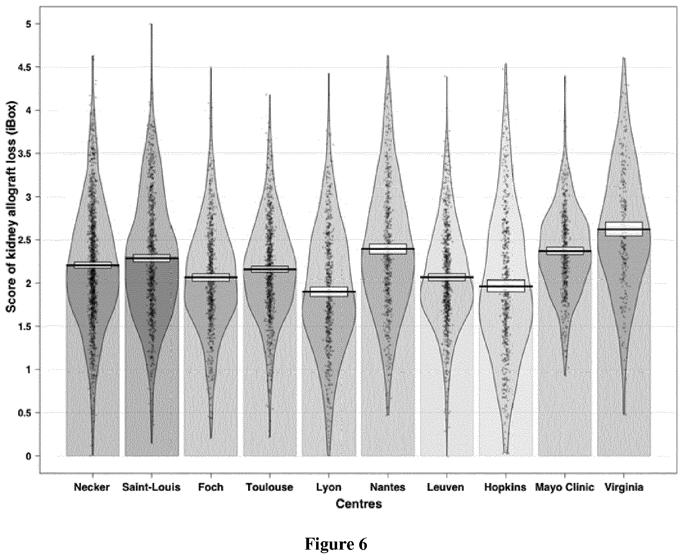

We then used several independent validation cohorts and confirmed the transportability of the iBox risk score in these geographically distinct cohorts. Overall, we demonstrated good discrimination performance in the external validation cohorts with a C statistic of 0.81 in Europe (95% bootstrap percentile CI=0.78 to 0.84) and 0.80 in the US (95% bootstrap percentile CI=0.76 to 0.84). The calibration plots showed optimal agreement between the iBox risk score-predicted probabilities of allograft survival at 3, 5 and 7 years after risk evaluation and actual kidney allograft survival (**Figure 2**). The distribution and density of the iBox risk prediction scores in each centre are shown in **Figure 6.**

*Performance of the iBox risk prediction score in therapeutic randomised controlled clinical trials*

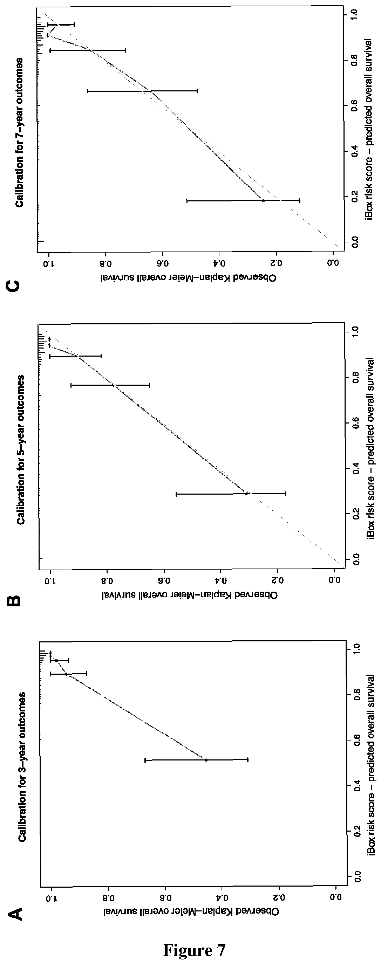

We tested the performance of the iBox risk prediction score in 3 registered and published phase II and III clinical trials.(20-22). The details of the clinical trials depicting the population, intervention, clinical scenario and follow-up times are presented in **Table 4.** We calculated the iBox risk prediction scores of all patients included in the trials and compared those with the actual allograft failures. The iBox risk prediction score applied in the three trials revealed accurate discrimination overall (C-index 0.87; 95% bootstrap percentile CIs=0.82 to 0.92). The calibration plot showed an optimal agreement between the risk prediction score based on predicted allograft loss and the actual observations of kidney allograft loss (**Figure 7**).

*Sensitivity analyses*

Various sensitivity analyses were performed to test the robustness and generalisability of the iBox risk score in different clinical scenarios and subpopulations.

*Added value of the iBox integrative risk prediction score compared to conventional allograft function monitoring (eGFR / proteinuria) and generation of an abbreviated functional iBox score.*

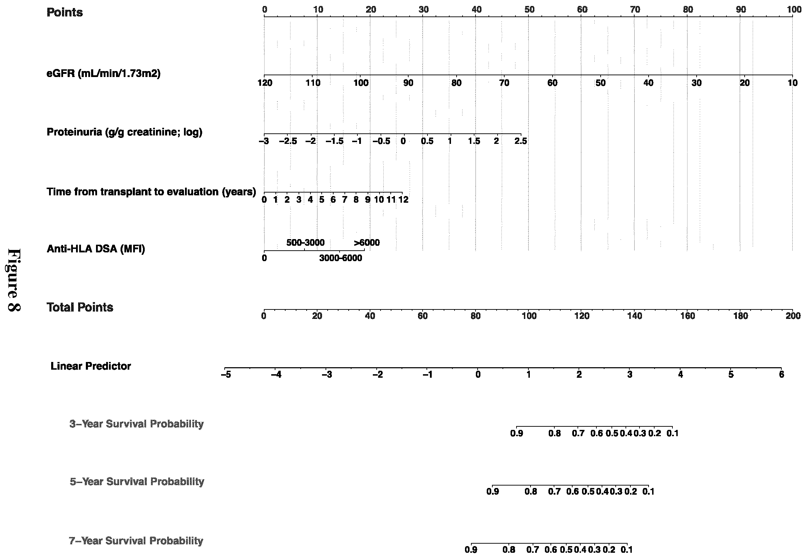

We tested the added value of the iBox risk prediction score over the conventional allograft monitoring model based on eGFR and proteinuria assessments. We demonstrated that the iBox risk score was superior in terms of prediction capability than a restricted model including eGFR and proteinuria (C-index=0.73; 95% bootstrap percentile CI=0.71 to 0.75, p-value <0.0001 as compared with the full iBox model). This was further demonstrated by a continuous net reclassification improvement (cNRI) of 0.228 for the iBox model compared to that of the functional model (95% CI, 0.174 to 0.290, p<0.0001). To account for potentially different medico-economic contexts limiting the availability of allograft biopsies, we are providing in this study an abbreviated iBox score based on clinical-functional parameters (**Figure 8**).

*Added value of the iBox risk prediction score compared to risk scores previously reported in the literature*

We performed a systematic review (see **EXAMPLE 3**) and compared the iBox risk prediction score to previously published risk scores assessing long-term allograft outcomes and demonstrated that the iBox prediction score outperformed other risk scores (see **EXAMPLE 3**).

*Prediction model performance using histological diagnoses instead of the Banff international classification histological lesion grading*

When histological diagnoses were included in the multivariable model instead of histological lesions graded according to the international Banff classification, antibody-mediated rejection (AMR) (p<0.001), T-cell mediated rejection (TCMR, p=0.045), primary nephropathy recurrence (p=0.003) and BK virus nephropathy (BKVAN, p=0.050) showed significant and independent associations with allograft failure. In this model, the set of non-histological predictors of allograft failure identified in the primary analyses remained unchanged (hazard ratios are shown for each parameter in **Table 10**). The discrimination ability of the histological diagnosis-based model revealed a C-index of 0.76 (95% bootstrap percentile CI=0.74 to 0.81).

*iBox performance when applied at the time of clinically indicated biopsies vs protocol biopsies*

We tested and confirmed the performance of the iBox risk prediction score when risk evaluation started at the time of clinically indicated allograft biopsies performed at any time after transplantation (n=1,598, 40%), as well as at the time of 1-year protocol biopsies (n=2,402, 60%; **Table 3**).

Similarly, the iBox risk score demonstrated accurate discrimination ability for long term allograft loss when risk evaluation started before 1-year post transplant or after 1-year post transplant (average post-transplantation time of 0.89±0.23 years and 2.31±1.66 years respectively; **Table 3**).

*IBox assessed in other clinical scenarios and subpopulations*

Finally, we confirmed the performance of the iBox risk prediction score when applied in different subpopulations and clinical scenarios including *i)* living and deceased donors, *ii)* according to recipient's ethnicity, *iii)* in highly sensitised (high immunological risk) and non-highly sensitised (low immunological risk) recipients, and iv) patient receiving an induction by anti-iL2 receptor or anti-thymocyte globulin (**Table 3**). When parameters assessed at the time of transplant (such as HLA mismatches), recipient blood pressure at the time of risk assessment (log scale), and calcineurin inhibitor through blood level at the time of risk assessment were forced in the risk prediction score, there was no significant improvement in its prognostic performance (**Table 3**).

### Discussion:

The iBox, a risk prediction score combining allograft functional, histological, and immunological parameters together with HLA antibody profiling, showed good performance in predicting the risk of long-term kidney allograft failure. We demonstrated the generalisability of the iBox risk prediction score by showing its external validity in six geographically distinct cohorts recruited in Europe and in the US with distinct allocation systems, patient characteristics and management practices. The iBox risk prediction score also demonstrated its accuracy when measured at different times post-transplantation, which permits to update the score based on new events that patient might encounter in their long-term course. We also demonstrated the added value of the iBox risk prediction score over a conventional allograft monitoring model that includes eGFR and proteinuria assessment and showed that the iBox risk prediction score outperformed other available risk scores applied in kidney transplant patients. Last, we confirmed the predictive accuracy of the risk score in the data issued from three published randomised therapeutic trials covering different clinical scenarios encountered after transplantation, further enhancing its value as a potential surrogate endpoint in transplantation.(20-22) Overall, the predictor variables used in the iBox risk prediction score are easily available after transplantation in most centres worldwide, making it feasible for implementation in routine clinical practice. To account for potential different medico-economic contexts limiting the availability of allograft biopsies, we also provide in this study an abbreviated score based on clinical-functional parameters.

Current prognostic scores implemented in clinical practice in transplant medicine mostly address the prediction of allograft survival at the time of transplantation; thus, their use is limited to allograft allocation because they do not inform posttransplant clinical decision making and patient monitoring.(31) The few attempts of developing posttransplant prognostic scores have failed to provide useful tools for transplant clinicians. According to a systematic review without date restrictions for publications up to July 25, 2018, for allograft survival scoring systems among kidney transplant recipients (see **EXAMPLE 3**), no study has developed and externally validated a posttransplant prognostic score usable at any time after transplantation. The main limitations to achieve a robust and validated scoring system rely on multiple factors including the insufficient data quality of the previously studied cohorts and the fact that no registry or database system has been primarily designed to address the specific aspect of prognostication. An even more important aspect is external validation in different populations, which prompted us to conduct a large external validation from multiple centres worldwide. Despite some expected loss of discriminative performance, models are typically considered useful for clinical decision making when the C-statistic is greater than 0.70 and strong when the C-statistic exceeds 0.80, suggesting that the iBox risk prediction score could support decision making.(32) Compared to prognostication systems in other fields such as oncology (e.g., locally advanced pancreatic cancer and metastatic colonic cancer), the C-index is typically closer to 0.60 or 0.70.(33) Taken together, these results confirm not only the robustness and validity of the iBox risk prediction score but also its generalisability to other transplant cohorts with different kidney allocation systems, donor and recipient profiles, and distinct patient management and healthcare environments.

In this study, we demonstrated that the iBox risk prediction score outperformed the current gold standard (eGFR and proteinuria) for the monitoring of kidney recipients. In particular, compared to prior attempts at developing a prognostication system, we found that allograft histological lesions such as microcirculation inflammation, interstitial inflammation-tubulitis (reflecting active rejection process) and atrophy-fibrosis, and transplant glomerulopathy (reflecting chronic allograft damage), in addition to measuring allograft functional parameters and recipient antibody profiles, improved the overall discrimination capacity of the model and that a multidimensional risk prediction score performs better than its individual components. This risk prediction score reflects the main patterns of allograft deterioration leading to failure, represented by alloimmune processes and allograft scarring.(34) Two other prognostic scores have attempted to combine several transplant diagnostic dimensions, including allograft function and pathology and alloantibodies; however, these scores were outperformed by the iBox risk prediction score.(16, 35)

Importantly, our results and the parameters included in the final model reinforce the potential of the iBox to be implemented into contemporary clinical practice by using automated approaches within electronic medical record systems (an online available electronic risk calculator is provided at http://www.paristransplantgroup.org and examples are provided in

**EXAMPLE 4**).

In addition, the combination of major drivers of allograft failure in the iBox risk prediction score were allowed to evaluate early the effect of clinical interventions on long-term allograft outcomes. In this study, we tested and validated the iBox risk prediction score in the setting of therapeutic clinical trials covering different clinical scenarios and demonstrated accurate performance overall. We found that the prediction of allograft failure assessed by the iBox score accurately fits with the actual graft failures observed in these trials at 5 years after risk evaluation. Importantly, the accuracy of the iBox risk prediction score was conserved regardless of the therapeutic intervention and population from those trials, with accurate performance in the *Certitem* (NCT01079143) calcineurin inhibitor minimisation trial (22) and rejection treatment trials (EudraCT 2007-003213-13; NCT01873157).(20, 21) This finding reinforced the potential of the iBox risk prediction score for defining a valid surrogate endpoint. Indeed, in the present study, a well-validated, strong and robust association existed between the surrogate and the true endpoint, and this association was consistent across different treatment settings.

Regarding the limitations of this study, we acknowledge that emerging predictors posttransplant might be missing in our model. Despite the already high performance achieved by the iBox risk prediction score, future studies should evaluate the added value of new non-invasive biomarkers or genetic factors in addition to those presently reported regarding discriminative capability, generalisability and overcoming the need for an invasive procedure (kidney allograft biopsy). Another limitation is that information regarding the drug adherence of single patients was lacking in our dataset. Although nonadherence is a major risk factor for graft failure, it is inherently difficult to capture, especially at a population level.(34) Notwithstanding that the iBox risk prediction score was primarily generated using a large, prospective, unselected cohort, a prospective validation of the iBox in daily clinical practice remains desirable. Finally, despite the validation of the iBox risk prediction score in an interventional setting, future trials are needed to compare whether a strategy based on a systematic risk evaluation vs. an empirical approach might improve clinical management.

**Conclusions:**

We developed and validated the iBox risk prediction score, which accurately predicts allograft failure after kidney transplantation. We demonstrated its generalisability and transportability across centres worldwide and its performance in therapeutic clinical trials. The iBox risk prediction score provides an accurate but simple strategy that can be easily implemented to stratify patients into clinically meaningful risk groups and that can be time-updated after transplant which may help guide patient monitoring in everyday practice and stratify patients in future clinical trials.

**EXAMPLE 2: SUPPLEMENTARY METHODS**

***Data collection procedures***

All data from Paris-Necker, Paris-Saint Louis, Foch and Toulouse hospitals were extracted from the prospective Paris Transplant Group Cohort data cohort (CNIL, Registration number: 363505, validated on the 8th of June 2004). The database networks have been approved by the National French Commission for bioinformatics data and patient liberty and codes were used to ensure strict donor and recipient anonymity and blind access. Informed consent was obtained from the participants at the time of transplantation. The data are computerised in real time and at the time of transplantation, at the time of post-transplant allograft biopsies and at each transplant anniversary and are submitted for an annual audit.

***Independent validation cohorts***

In the European validation cohort, the French data from the Lyon, and Nantes Hospitals for donors and recipients were extracted from the DIVAT clinical prospective cohort (official website: www.divat.fr) and from the French national agency database CRISTAL (official website: https://www.sipg.sante.fr/portail/). The Belgian data and data from the North-American validation cohort were collected as part of routine clinical practice and entered in centres' databases in compliance with local and national regulatory requirements. They were sent anonymised to the Paris Transplant Group.

***Prognostic parameters prospectively collected and assessed in the derivation cohort***

Baseline recipient's characteristics:

1. Recipient's age
2. Recipient's gender
3. Recipient's height
4. Recipient's weight
5. Previous transplantation
6. Delay between dialysis and transplantation
7. Cause of end stage renal disease
8. ABO blood group
9. HLA genotype
10. CMV serology
11. HCV serology
12. HBV serology
13. HIV serology

Baseline donor's characteristics:

14. Donor's age
15. Donor's gender

16. Donor's height
17. Donor's weight
18. Type of donor: deceased vs living
19. Cause of donor's death
20. Double transplantation
21. History of hypertension
22. History of diabetes
23. ECD status
24. Serum creatinine
25. ABO blood group
26. HLA genotype
27. CMV serology
28. HCV serology
29. HBV serology
30. HIV serology

Immunological characteristics at the time of transplantation:

31. HLA mismatches A
32. HLA mismatches B
33. HLA mismatches Cw
34. HLA mismatches DQ
35. HLA mismatches DR
36. HLA mismatches DP
37. Anti-HLA DSA at the time of transplantation
38. MFI of the anti-HLA DSA at the time of transplantation
39. cPRA

Transplant characteristics:

40. Cold ischemia time
41. Delayed graft function
42. Induction treatment with anti-thymocyte globulin
43. Induction treatment with basiliximab
44. Steroid dose

Immunological data at the time of risk assessment (Luminex SA assessment A. B, C, DP, DQ, DR)

45. Anti-HLA DSA
46. MFI of immunodominant anti-HLA DSA

Histological data according to the Banff classification:

47. g Banff score
48. ptc Banff score
49. t Banff score
50. i Banff score
51. cg Banff score
52. v Banff score
53. mm Banff score
54. ci Banff score
55. ct Banff score

56. IFTA Banff score
57. cv Banff score
58. ah Banff score
59. C4d ptc deposition
60. Recurrence of ESRD
61. Polyomavirus-associated nephropathy
62. ABMR status
63. TCMR status
64. Borderline category

Follow-up variables:

65. Episodes of pyelonephritis
66. Immunosuppression treatment
67. Type of treatment: calcineurin inhibitors, mycophenolate mofetil, mTOR inhibitors or belatacept
68. CNI blood through level at M12 and every year
69. Steroid dose at M12 and every year
70. Rejection therapy (e.g., steroid, plasma exchange, intravenous immunoglobulin)
71. CMV prophylaxis
72. BK viral load at M12 and every year
73. CMV viral load at M12 and every year
74. Allograft function at M12 and every year
75. Proteinuria at M12 and every year
76. Patient date and cause of allograft loss
77. Patient date and cause of death

### Detection and Characterisation of Donor-specific Anti-HLA Antibodies

All patients were tested for the presence of circulating anti-HLA donor-specific antibodies (DSAs) at the time of patient risk evaluation. The presence of circulating DSAs against HLA-A, HLA-B, HLA-Cw, HLA-DR, HLA-DQ and HLA-DP was retrospectively determined using single-antigen flow bead assays (One Lambda, Inc., Canoga Park, CA, USA) on a Luminex platform. Beads with a normalised mean fluorescence intensity (MFI), a measure of donor-specific antibody strength, of greater than 500 units were judged as positive as previously described. HLA typing of the transplant recipients and donors was performed using an Innolipa HLA Typing Kit (Innogenetics, Ghent, Belgium). In the validation cohorts, HLA genotyping and HLA antibody profiling were performed according to local centre practice.

### Kidney Allograft Phenotypes at time of risk assessment

In the derivation cohort, allograft biopsies were scored and graded from 0 to 3 according to the updated Banff criteria for allograft pathology for the following histological factors: glomerular inflammation (glomerulitis), tubular inflammation (tubulitis), interstitial inflammation, endarteritis, peritubular capillary inflammation (capillaritis), transplant glomerulopathy, interstitial fibrosis, tubular atrophy, arteriolar hyalinosis and arteriosclerosis. Additional diagnoses provided by the biopsy (e.g., the diagnoses of primary disease recurrence, BK virus nephropathy) were recorded. The biopsy sections (4 $\mu$m) were stained with periodic acid-Schiff, Masson's trichrome, and hematoxylin and eosin. C4d staining was performed via immunohistochemical analysis on paraffin sections using polyclonal human anti-C4d antibodies. Also, in the validation cohorts, the Banff criteria for the individual histological lesions were assessed in each biopsy included in the study.

### Statistical analysis interpretation

#### Continuous variables

When used as continuous variables in the Cox model, a potential non-linear relationship between predictors and allograft loss was first investigated using restricted cubic splines modelling. Secondly, a fractional polynomial method was applied to determine the best transformation for continuous variables. For donor age, recipient age, eGFR and HLA mismatches, a linear relationship with outcome was found to be a good approximation. A logarithmic transformation was necessary for proteinuria and time post-transplant.

*Discrimination*

The aim of discrimination is to distinguish between patients who experience an event and those who do not. The C-index estimates the proportion of all pairwise patient combinations from the sample data whose survival time can be ordered such that the patient with the highest predicted survival is the one who actually survived longer (discrimination). The C-index ($0 \leq C \leq 1$) is the probability of concordance between predicted and observed survival, with C-index=0.5 for random predictions and C-index=1 for a perfectly discriminating model.

*Calibration*

Calibration refers to the ability to provide unbiased survival predictions in groups of similar patients. It estimates how close the score-estimated risk is to the observed risk. A prediction model is considered "well calibrated" if the difference between predictions and observations in all groups of similar patients is close to 0 (perfect calibration). Any large deviation ($p<0.1$) indicates a lack of calibration.

*Bootstrapping*

Bootstrapping is the preferred simulation technique that was first described by Bradley Efron. The original dataset is a random sample of patients being representative of a general population. Bootstrapping means generating a large number of datasets, each of which with the same sample size as the original one, by resampling with replacement (i.e., a previously selected patient may be selected again).

*Internal validation*

Internal validation is useful to obtain an honest estimate of the model performance for patients who are similar to those in the development sample and to indicate an upper limit to the expected performance in other settings. The bootstrap approach is the preferred technique to assess internal validity. The internal validity of the final model was confirmed using a bootstrap procedure, which involved generating 1,000 datasets derived from resampling the original dataset and permitted the estimation of the bias-corrected 95% CI and the accelerated bootstrap (BCA) HR.

*External validation*

External validation may show different results from internal validation since many aspects may be different between settings, including selection of patients, definitions of variables, and diagnostic or therapeutic procedures. The strength of the evidence for the score validity is usually considered greater with a fully external validation (e.g., other investigators and centres).

*Competing risk by death analysis*

We estimated cumulative incidence functions from competing risks data and compared the subdistribution for each cause across groups. We then assessed the effects of predictive factors (iBox risk strata) on the subdistribution of graft loss in a competing risks setting with death by fitting the proportional subdistribution hazard regression model described in the Fine and Gray method.

**Construction of the integrative score derived from the final multivariable Cox model**

$$\text{Raw Prognostic score}_{\text{Cox model}} = \begin{aligned} &\text{Estimated GFR} \times \alpha \\ &+ \text{Proteinuria (log transformation)} \times \beta \\ &+ \text{Interstitial fibrosis and tubular atrophy } 2 \binom{0}{1} \times \chi \\ &+ \text{Interstitial fibrosis and tubular atrophy } 3 \binom{0}{1} \times \chi' \\ &+ \text{Microvascular inflammation (g + ptc) } 3 - 4 \binom{0}{1} \times \delta \\ &+ \text{Microvascular inflammation (g + ptc) } 5 - 6 \binom{0}{1} \times \delta' \\ &+ \text{Anti} - \text{HLA DSA MFI} \quad \text{MFI } 500 - 3000 \binom{0}{1} \times \varepsilon \\ &+ \text{Anti} - \text{HLA DSA MFI} \quad \text{MFI } 3000 - 6000 \binom{0}{1} \times \varepsilon' \\ &+ \text{Anti} - \text{HLA DSA MFI} \quad \text{MFI} > 6000 \binom{0}{1} \times \varepsilon'' \\ &+ \text{Transplant glomerulopathy } \binom{0}{1} \times \partial \\ &+ \text{Interstitial inflammation and tubulitis (i + t) } 3 - 6 \binom{0}{1} \times \mu \\ &+ \text{Time from transplant to Risk evaluation} - \text{year} \times \phi \end{aligned}$$

$\alpha, \beta, \chi, \delta, \partial, \mu, \varepsilon$ and $\phi$ : Cox-model beta coefficients for the corresponding parameters

$$\text{Raw Prognostic score}_{\text{Cox model}} \in [\min\_score - \max\_score]$$

$$\text{Normalized Prognostic score}_{\text{Cox model}} = \frac{(5 - 0) \times (\text{Raw Prognostic score}_{\text{Cox model}} - \min\_score)}{\max\_score - \min\_score} + 0$$

$$\text{Normalized Prognostic score}_{\text{Cox model}} \in [0 - 5]$$

**EXAMPLE 3: ADDED VALUE OF THE IBOX RISK PREDICTION SCORE COMPARED TO RISK SCORES PREVIOUSLY REPORTED IN THE LITERATURE**

A comprehensive search strategy was conducted through several databases (PubMed, Medline, Embase, Cochrane, and Scopus) without date restrictions for publications up to July 25, 2018 for allograft survival scoring systems among kidney transplant recipients. We used the search terms "kidney transplantation", "allograft survival" and "prognostic score". Out of 460 articles identified, 11 were related to long-term allograft survival, 5 were externally validated and only 2 comprised immunological parameters. They are presented in **Table 9** and compared with the iBox risk prediction score. The two studies identified: i) were not derived from patient cohorts with systematic monitoring and specific design towards risk stratification; ii) did not integrate a large spectrum of potential prognostic factors, iii) were not validated in multiple large cohorts worldwide with different transplant allocation systems and management practices, iv) were not validated in randomised controlled therapeutic clinical trials (RCTs).

**EXAMPLE 4: IBOX PRACTICAL APPLICATION FOR CLINICIANS: READY-TO-USE INTERFACE FOR CLINICIANS.**

Real-life patients for whom we used the iBox risk score to predict individual 3, 5 and 7-year- allograft survival. Patients #1 to #3 were from the iBox database reference set. Patient #4 was from the randomized controlled trial: RITUX ERAH Eudra CT 2007-003213-13.

*Patient #1 description*

A 64-year-old male with membranoproliferative glomerulonephritis underwent a second preemptive kidney transplantation from an expanded-criteria deceased donor in 2013. The patient was sensitised (cPRA of 50%) without circulating anti-HLA DSA identified at the time of transplantation. Initial immunosuppressive regimen included anti-thymocyte globulin induction with corticosteroids, mycophenolate mofetil and tacrolimus.

Three months after transplantation, eGFR (MDRD) was 52 mL/min/1.73 m$^2$ without proteinuria (0.05 g/g). The evaluation at one-year post-transplantation found an eGFR (MDRD) of 33 mL/min/1.73 m$^2$. No circulating anti-HLA DSA nor proteinuria were detected. The biopsy revealed severe interstitial fibrosis and tubular atrophy was detected (IFTA Banff score=3) as well as a glomerulitis (g score=1). No other lesion was observed (ptc, c4d, cg, i, t scores=0).

$$\text{iBox}_{\text{Patient \#1}} = \beta_{\text{time from transplant to risk evaluation}} * 3 + \beta_{\text{eGFR}} * 33 + \beta_{\text{Proteinuria}} * \log(0.05) +$$

$$\beta_{\text{DSA MFI}} * 0 + \beta_{\text{g+ptc}} * 1 + \beta_{\text{i+t}} * 0 + \beta_{\text{cg}} * 0 + \beta_{\text{IFTA}} * 3$$

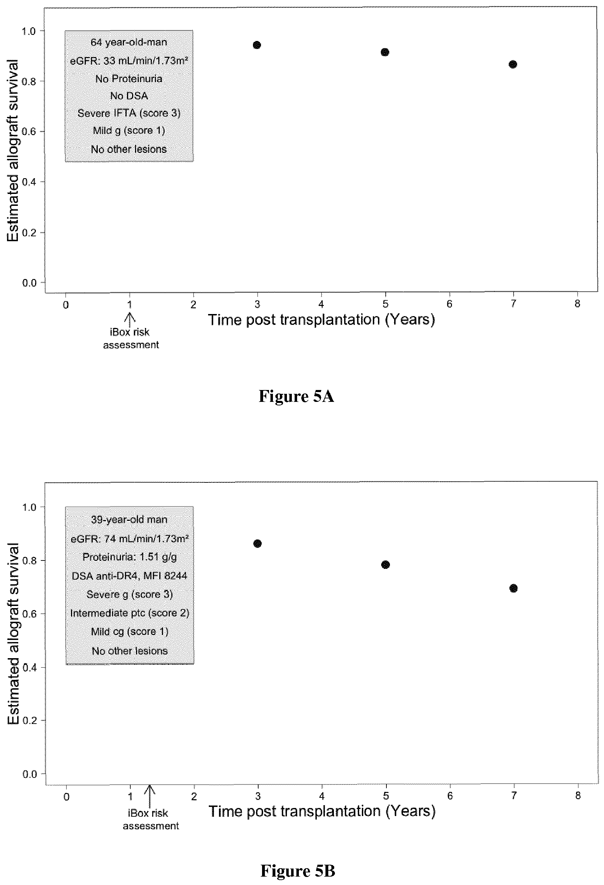

Patient #1 individual allograft survival probabilities at 3, 5 and 7-years are 94%, 91%, and 86% respectively (see **Figure 5A**: iBox report of allograft survival projection).

***Patient #2 description***

A 39-year-old male patient with an obstructive uropathy underwent a first living-related donor kidney transplantation in 2012, with a cPRA at 0 at the time of transplantation. The immunosuppressive regimen consisted in basiliximab, corticosteroids, mycophenolate mofetil and tacrolimus.

At 15 months, the patient developed a *de novo* DSA (anti-DR4, MFI 8,244). At the time of dnDSA identification, the eGFR (MDRD) was 74 mL/min/1.73 m$^2$ with a proteinuria of 1.51 g/g. A biopsy was performed with a g score=3, ptc score=2, C4d score=2, and transplant glomerulopathy score=1. No other lesion was observed (IFTA, i, t scores = 0).

$$\text{iBox}_{\text{Patient\#2}} = \beta_{\text{time from transplant to risk evaluation}} * 15 + \beta_{\text{eGFR}} * 74 + \beta_{\text{Proteinuria}} * \log(1.51) +$$

$$\beta_{\text{DSA MFI}} * 3 \text{ (e.g. greater than 6'000 of MFI)} + \beta_{\text{g+ptc}} * 5 + \beta_{\text{i+t}} * 0 + \beta_{\text{cg}} * 1 + \beta_{\text{IFTA}} * 0$$

The iBox score projects the patient in the strata 3. The 3, 5 and 7-year probabilities of allograft survival are 86%, 78%, and 69% respectively (see **Figure 5B**: iBox report of allograft survival projection).

***Patient #3 description***

A 45-year-old woman with an end-stage renal disease due to a type 1 diabetes underwent her first kidney transplantation with a standard criteria deceased donor in 2009. She was highly sensitised due to blood transfusions (cPRA=89%) but without detectable circulating anti-HLA DSA. Immunosuppressive treatment included an induction therapy with anti-thymocyte globulin and a maintenance immunosuppressive regimen of corticosteroids, MMF and tacrolimus.

At 5 months post-transplant, eGFR (MDRD) was 62 mL/min/1.73 m$^2$, a *de novo* DSA was detected (anti-DQ8, MFI 1,233) and a proteinuria was identified (0.19 g/g). An allograft biopsy revealed a mild interstitial fibrosis/tubular atrophy (IFTA score=1). The biopsy was free from other pathological lesion (g, ptc, c4d, cg, i, t Banff scores=0).

$$\text{iBox}_{\text{Patient \#3, 1st score}} = \beta_{\text{time from transplant to risk evaluation}} * 5 + \beta_{\text{eGFR}} * 62 + \beta_{\text{Proteinuria}} * \log(0.19)$$

$$+ \beta_{\text{DSA MFI}} * 1 \text{ (e.g. between 500 and 3'000 of MFI)} + \beta_{\text{g+ptc}} * 0 + \beta_{\text{i+t}} * 0 + \beta_{\text{cg}} * 0 + \beta_{\text{IFTA}} * 1$$

The patient #3 individual allograft survival probabilities at 3, 5 and 7 years are 97 %, 96 %, and 94 % respectively.

The same patient was then reevaluated 13 months post transplantation with a decreased eGFR at 43 ml/min/1.73 m$^2$. The MFI of the anti-DQ8 *dn*DSA increased to 7'358. A new biopsy was performed showing a transplant glomerulopathy (cg score of 1). The other parameters were stable otherwise, when compared with the previous biopsy.

$$iBox_{\text{Patient \#3, 2nd score}} = \beta_{\text{time from transplant to risk evaluation}} * 13 + \beta_{\text{eGFR}} * 43 + \beta_{\text{Proteinuria}} * \log(0.22)$$

$$+ \beta_{\text{DSA MFI}} * 3 \text{ (e.g. greater than 6'000)} + \beta_{\text{g+ptc}} * 0 + \beta_{\text{i+t}} * 0 + \beta_{\text{cg}} * 1 + \beta_{\text{IFTA}} * 1$$

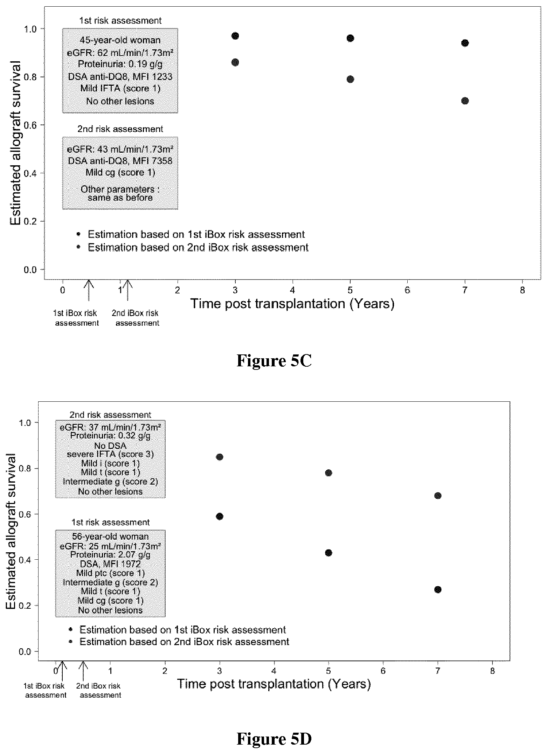

The iBox prediction score for patient #2 is updated with 86%, 78%, and 68% individual allograft survival probabilities at 3, 5 and 7-years to respectively (see **Figure 5C**:).

***Patient #4 description (Rituxerah trial Eudra CT 2007-003213-13)***

A 56-year-old woman with tubulointerstitial nephropathy underwent a first kidney transplantation in 2011 (standard criteria deceased donor). At Day 0 no circulating anti-HLA DSA was detected and an induction with anti-thymocyte globulin was followed by an immunosuppression with corticosteroids, mycophenolate mofetil and calcineurin inhibitor. After 10 days, GFR was estimated at 48 mL/min/1.73 m$^2$ without proteinuria.

At month 1 post-transplant, the patient presented with a decreased allograft function; eGFR of 25 mL/min/1.73 m$^2$, a circulating *de novo* DSA (anti-B44, MFI 1,972), and a proteinuria of 2.07 g/g. A biopsy was performed and found an active ABMR (g2, ptc1, c4d3 according to Banff scoring system), with mild tubulitis (t score 1) and arteriolar hyalinosis (ah score 1). She was included in Rituxerah trial Eudra CT 2007-003213-13 in the placebo group (plasma exchange, intravenous immunoglobulin and steroid according to the protocol).

Below is the IBox evaluation at the time of patient inclusion:

$$IBox_{\text{Patient \#4, 1st score}} = \beta_{\text{time from transplant to risk evaluation}} * 1 + \beta_{\text{eGFR}} * 25 + \beta_{\text{Proteinuria}} * \log(2.07)$$

$$+ \beta_{\text{DSA MFI}} * 1 \text{ (e.g. between 500 and 3000 of MFI)} + \beta_{\text{g+ptc}} * 3 + \beta_{\text{i+t}} * 1 + \beta_{\text{cg}} * 0 + \beta_{\text{IFTA}} * 0$$

The patient #4 individual allograft survival probabilities at the time of the therapeutic intervention were 59 %, 43 %, and 27 % at 3, 5 and 7 years, respectively.

Six months after inclusion, the eGFR was of 37 mL/min/1.73 m$^2$, proteinuria was 0.32 g/g of creatininuria and the previously identified anti-HLA DSA was undetectable. The biopsy found an acute borderline T-cell mediated rejection according to the Banff classification (i score 1 and t score 1), arteriosclerosis (cv score 1), mild arteriolar hyalinosis (ah score 1), glomerulitis score of 2 and interstitial fibrosis and tubular atrophy (IFTA score 3).

$$IBox_{\text{Patient \#4, 2nd score}} = \beta_{\text{time from transplant to risk evaluation}} * 7 + \beta_{\text{eGFR}} * 37 + \beta_{\text{Proteinuria}} * \log(0.32)$$

$$+ \beta_{\text{DSA MFI}} * 0 + \beta_{\text{g+ptc}} * 2 + \beta_{\text{i+t}} * 2 + \beta_{\text{cg}} * 0 + \beta_{\text{IFTA}} * 3$$

The IBox score after therapeutic intervention now projects the patient survival to updated 3, 5 and 7 year-allograft survival probabilities of 85 %, 78 %, and 68 % respectively (see **Figure 5D**: iBox report of allograft survival projection).

**TABLES:**

Table 1: Patient characteristics by cohort

| | Derivation cohort (n=4,000) | | European validation cohort (n=2,129) | | North American validation cohort (n=1,428) | | p* |
|---|---|---|---|---|---|---|---|
| | | n | | n | | n | |
| **Recipient demographics** | | | | | | | |
| **Age** (years), mean (SD) | 4,000 | 49.83 (13.70) | 2,129 | 50.58 (13.66) | 1,420 | 50.42 (14.17) | 0.0916 |
| **Gender male**, No. (%) | 4,000 | 2,450 (61.25) | 2,129 | 1,333 (62.61) | 1,428 | 830 (58.12) | **0.0250** |

(continued)

| | Derivation cohort (n=4,000) | | European validation cohort (n=2,129) | | | North American validation cohort (n=1,428) | p* |
|---|---|---|---|---|---|---|---|
| | | n | | n | n | | |
| **Recipient demographics** | | | | | | | |
| **End-stage renal disease causes** | 4,000 | | 2,129 | | 1,428 | | |
|   **Glomerulonephritis**, No. (%) | | 1,086 (27.15) | | 584 (27.43) | | 365 (25.56) | |
|   **Diabetes,** No. (%) | | 438 (10.95) | | 316 (14.84) | | 271 (18.98) | |
|   **Vascular**, No. (%) | | 296 (7.40) | | 139 (6.53) | | 249 (17.44) | |
|   **Other**, No. (%) | | 2,180 (54.50) | | 1,090 (51.20) | | 543 (38.03) | **<0.0001** |
| **Transplant characteristics** | | | | | | | |
| **Donor age** (years), mean (SD) | 4,000 | 51.68 (16.33) | 2,122 | 48.24 (15.79) | 1,420 | 41.01 (14.75) | **<0.0001** |
| **Donor male gender**, No. (%) | 4,000 | 2,151 (53.78) | 2,124 | 1,225 (57.67) | 1,420 | 694 (48.87) | **<0.0001** |
| **Donor hypertension**, No. (%) | 3,903 | 1,005 (25.75) | 1,876 | 450 (23.99) | 1,287 | 189 (14.69) | **<0.0001** |
| **Donor diabetes mellitus**, No. (%) | 3,861 | 231 (5.98) | 1,713 | 47 (2.74) | 1,276 | 47 (3.68) | **<0.0001** |
| **Donor serum creatinine >1.5 mg/dL**, No. (%) | 3,962 | 422 (10.65) | 1,936 | 193 (9.97) | 1,075 | 284 (26.42) | **<0.0001** |
| **Donor type** | | | | | | | |
| **Deceased donor**, No. (%) | 4,000 | 3,327 (83.18) | 2,129 | 1,974 (92.72) | 1,428 | 620 (43.42) | **<0.0001** |
| **Death from cerebrovascular disease**, No. (%) | 3,327 | 1,864 (56.03) | 1,974 | 993 (50.30) | 618 | 194 (31.39) | **<0.0001** |
| **Expanded criteria donor**, No. (%) | 3,995 | 1,409 (35.27) | 2,010 | 628 (31.24) | 1,425 | 72 (5.05) | **<0.0001** |
| **Prior kidney transplant**, No. (%) | 4,000 | 605 (15.13) | 2,129 | 322 (15.12) | 1,408 | 235 (16.69) | **0.3410** |
| **Cold ischemia time** (hours), mean (SD) | 3,976 | 16.20 (8.99) | 2,093 | 15.50 (7.30) | 1,212 | 9.51 (11.81) | **<0.0001** |
| **Delayed graft function[†]**, No. (%) | 3,897 | 1,046 (26.84) | 2,127 | 476 (22.38) | 1,424 | 158 (11.10) | **<0.0001** |
| **HLA-A/B/DR mismatch**, mean (SD), number | 4,000 | 3.817 (1.36) | 2,083 | 3.15 (1.39) | 1,427 | 3.54 (1.79) | **<0.0001** |

Abbreviations: ESRD: end-stage renal disease; HLA: human leucocyte antigen.

* p-value is based on a comparison of all cohorts.

† Delayed graft function was defined as the use of dialysis in the first postoperative week.

**Table 2A: Factors assessed at the time of posttransplant risk evaluation associated with kidney allograft failure in the derivation cohort: univariable analysis**

| | | | Number of patients | Number of events* | HR | 95% CI | p |
|---|---|---|---|---|---|---|---|
| **Recipient characteristics** | **Age** (per 1-year increment) | | 4,000 | 549 | 1.002 | (0.996 to 1.009) | 0.4575 |
| | **Gender** | **Female** | 1,550 | 214 | 1 | - | |
| | | **Male** | 2,450 | 335 | 1.004 | (0.845 to 1.191) | 0.9675 |
| **Transplant characteristics** | **Donor age** (per 1-year increment) | | 4,000 | 549 | 1.016 | (1.011 to 1.022) | **<0.0001** |
| | **Donor gender** | **Female** | 1,849 | 254 | 1 | - | |

(continued)

| | | Number of patients | Number of events* | HR | 95% CI | p |
|---|---|---|---|---|---|---|
| | Male | 2,151 | 295 | 0.981 | (0.830 to 1.161) | 0.8257 |
| Donor type | Living | 673 | 51 | 1 | - | |
| | Deceased | 3,327 | 498 | 2.057 | (1.542 to 2.744) | <0.0001 |
| Donor hypertension | No | 2,898 | 340 | 1 | - | |
| | Yes | 1,005 | 195 | 1.841 | (1.543 to 2.195) | <0.0001 |
| Donor diabetes mellitus | No | 3,630 | 491 | 1 | - | |
| | Yes | 231 | 31 | 1.392 | (1.005 to 1.929) | 0.0467 |
| Creatinine | <1.5 mg/dL | 3,540 | 467 | 1 | - | |
| | ≥1.5 mg/dL | 422 | 75 | 1.429 | (1.120 to 1.824) | 0.0041 |
| Expanded criteria donor | No | 2,586 | 285 | 1 | - | |
| | Yes | 1,409 | 263 | 1.896 | (1.603 to 2.242) | <0.0001 |
| Prior kidney transplant | No | 3,395 | 421 | 1 | - | |
| | Yes | 605 | 128 | 1.863 | (1.528 to 2.270) | <0.0001 |
| Cold ischemia time | <12 hours | 1,120 | 106 | 1 | - | |
| | 12-24 hours | 2,099 | 319 | 1.614 | (1.296 to 2.011) | |
| | ≥24 hours | 757 | 121 | 1.731 | (1.334 to 2.247) | <0.0001 |
| Thymoglobulin induction | No | 1,643 | 109 | 1 | - | |
| immunosuppression | Yes | 2,104 | 316 | 1.252 | (1.051 to 1.491) | 0.0118 |
| No. of HLA-A/B/DR mismatches | | 4,000 | 549 | 1.034 | (0.972 to 1.100) | 0.2939 |
| Preexisting anti-HLA donor-specific | No | 3,278 | 425 | 1 | - | |
| antibody | Yes | 722 | 124 | 1.510 | (1.234 to 1.844) | 0.0001 |

(continued)

| | | | Number of patients | Number of events* | HR | 95% CI | p |
|---|---|---|---|---|---|---|---|
| **Time of risk evaluation** | Time from transplant to evaluation (per 1-year increment) | | 3,996 | 549 | 1.264 | (1.205 to 1.325) | |
| **Functional** | **eGFR (mL/min/1.73 m$^2$)** | | 4,000 | 549 | 0.940 | (0.935 to 0.946) | |
| **parameters** | **Proteinuria at 1 year** (log transformation) | | 4,000 | 549 | 1.988 | (1.858 to 2.126) | |
| **Structural** | **Interstitial fibrosis /** | **0-1** | 3,099 | 331 | 1 | - | |
| **histopathology** | **tubular atrophy** | **2** | 555 | 116 | 2.149 | (1.739 to 2655) | |
| **parameters** | | **3** | 321 | 95 | 3.356 | (2.671 to 4.216) | |
| | **Arteriosclerosis** | **0** | 1,365 | 137 | 1 | - | |
| | | **≥1** | 2,446 | 386 | 1.619 | (1.332 to 1.967) | |
| | **Hyalinosis** | **0** | 1,567 | 149 | 1 | - | |
| | | **≥1** | 2,360 | 381 | 1.739 | (1.439 to 2.102) | |
| | **Interstitial inflammation** | **0-2** | 3,610 | 546 | 1 | - | |
| | **and tubulitis** | **≥3** | 390 | 93 | 1.969 | (1.575 to 2.460) | |
| | **Transplant** | **0** | 3,702 | 449 | 1 | | |
| | **glomerulopathy** | **≥ 1** | 260 | 94 | 3.701 | (2.962 to 4.624) | |
| | **Endarteritis** | **0** | 3,794 | 506 | 1 | - | |
| | | **≥1** | 96 | 27 | 2.263 | (1.537 to 3.333) | |
| | **C4d graft deposition** | **No** | 3,452 | 416 | 1 | - | |
| | | **Yes** | 548 | 133 | 2.446 | (2.011 to 2.976) | |
| | **Microcirculation inflammation** | **0-2** | 3,616 | 261 | 1 | | |
| | **(g+ptc)** | **3-4** | 308 | 92 | 3.069 | (2.448 to 3.846) | |
| | | **5-6** | 76 | 35 | 4.986 | (3.530 to 7.041) | |
| | **Polyomavirus associated** | **No** | 3,902 | 518 | 1 | - | |
| | **nephropathy** | **Yes** | 97 | 31 | 2.817 | (1.960 to 4.047) | |
| | **Nephropathy recurrence** | **No** | 3,868 | 510 | 1 | - | |
| | | **Yes** | 130 | 38 | 2.551 | (1.835 to 3.547) | |

(continued)

| | | | Number of patients | Number of events* | HR | 95% CI | p |
|---|---|---|---|---|---|---|---|
| | Antibody-mediated rejection | No | 3,398 | 368 | 1 | - | |
| | | Yes | 600 | 181 | 3.35 9 | (2.810 to 4.015) | |
| | T-cell-mediated rejection | No | 3,812 | 503 | 1 | - | |
| | | Yes | 187 | 46 | 1.96 4 | (1.452 to 2.656) | |
| Immunological | Anti-HLA donor-specific antibody | <500 | 3,312 | 394 | 1 | - | |
| parameters | mean fluorescence intensity | ≥500 3,000 | - | 483 | 82 | 1.66 3 | (1.310 to 2.111) |
| | | ≥3000 6,000 | - | 82 | 24 | 3.10 8 | (2.057 to 4.695) |
| | | ≥6,000 | | 123 | 49 | 4.55 7 | (3.383 to 6.138) |

* Number of events at 7 years post-iBox risk evaluation.

**Table 2B: Independent determinants of kidney allograft loss assessed at the time of posttransplant risk evaluation in the derivation cohort: multivariable analysis**

| | | Number of patients | Number of events* | 95% CI | p | Internal validation HR 95% CI bootstrap BCA |
|---|---|---|---|---|---|---|
| **Time from transplant to evaluation (years)** | | 3,941 | 538 | (1.023 to 1.138) | **0.0051** | (1.017 to 1.145) |
| **eGFR (mL/min/1.73 m$^2$)** | | 3,941 | 538 | (0.950 to 0.961) | **<0.0001** | (0.949 to 0.962) |
| **Proteinuria (log)** | | 3,941 | 538 | (1.398 to 1.628) | **<0.0001** | (1.384 to 1.640) |
| **Interstitial fibrosis/ tubular atrophy (IFTA)** | 0/1 | 3,074 | 330 | - | | - |
| | 2 | 550 | 115 | (0.918 to 1.424) | | (0.918 to 1.426) |
| | 3 | 317 | 93 | (1.083 to 1.773) | **0.0311** | (1.063 to 1.743) |
| **Microcirculation inflammation (g+ptc)** | 0-2 | 3,568 | 414 | - | | - |
| | 3-4 | 299 | 90 | (1.121 to 1.876) | | (1.099 to 1.899) |
| | 5-6 | 74 | 34 | (1.240 to 2.706) | **0.0010** | (1.207 to 2.799) |
| **Interstitial inflammation and tubulitis (i+t)** | 0-2 | 3,559 | 447 | - | | - |
| | ≥3 | 382 | 91 | (1.061 to 1.684) | **0.0136** | (1.031 to 1.712) |

(continued)

|  |  | Number of patients | Number of events* | 95% CI | p | Internal validation HR 95% CI bootstrap BCA |
|---|---|---|---|---|---|---|
| Transplant glomerulopathy (cg) | 0 | 3,684 | 445 |  |  | - |
|  | ≥ 1 | 257 | 93 | (1.133 to 1.895) | 0.0036 | (1.138 to 1.929) |
| Anti-HLA donor-specific antibody mean fluorescence intensity | <500 | 3,265 | 387 | - |  | - |
|  | ≥500 - 3,000 | 477 | 80 | (0.965 to 1.606) |  | (0.948 to 1.637) |
|  | ≥3,000 - 6,000 | 80 | 23 | (1.115 to 2.659) |  | (0.949 to 2.681) |
|  | ≥6,000 | 119 | 48 | (1.472 to 2.860) | 0.0001 | (1.484 to 2.879) |

The final multivariable Cox model was obtained by entering the risk factors from the univariable models that met p≤0.10 as the threshold in a single multivariable proportional hazards model. The final multivariable model was adjusted for the following parameters: expanded criteria donor (ECD), deceased donor, donor diabetes, cold ischemia time, thymoglobulin induction, circulating donor-specific anti-HLA antibody MFI at day 0, circulating donor-specific anti-HLA antibody MFI at the time of biopsy, cv Banff score, ah Banff score, i and t Banff scores, v score, cg Banff score, IFTA Banff score, microcirculation inflammation (g+ptc) score, C4d graft deposition, eGFR, proteinuria and the time of iBox evaluation.

Abbreviations: HR, hazard ratio; CI, confidence interval; BCA, bias-corrected and accelerated bootstrap; HLA, human leukocyte antigen.

* Number of events at 7 years post-iBox risk evaluation.

## Table 3: iBox risk prediction score performance when assessed in different clinical scenarios and subpopulations

| iBox risk score performance assessed in different clinical scenarios and subpopulations | Risk Model Performance (C-statistic) | 95% bootstrap percentile CIs |
|---|---|---|
| iBox using eGFR and proteinuria monitoring (without histology) | 0.79 | (0.77 to 0.81) |
| iBox using histology diagnoses* instead of Banff lesions grading | 0.76 | (0.74 to 0.81) |
| iBox in stable patients (protocol biopsy) | 0.81 | (0.77 to 0.86) |
| iBox in unstable patients (biopsy for cause) | 0.80 | (0.78 to 0.82) |
| iBox assessed in the first year after transplant | 0.77 | (0.72 to 0.81) |
| iBox assessed after 1-year post transplant | 0.84 | (0.82 to 0.87) |
| iBox in living donors | 0.82 | (0.75 to 0.88) |
| iBox in deceased donors | 0.80 | (0.78 to 0.82) |
| iBox in highly sensitised recipients † | 0.80 | (0.76 to 0.84) |
| iBox in non-highly sensitised recipients | 0.81 | (0.79 to 0.83) |
| iBox adding transplant baseline characteristics‡ | 0.81 | (0.79 to 0.83) |
| iBox in patient with anti-IL2 receptor induction | 0.79 | (0.76 to 0.82) |
| iBox in patients with anti-thymocyte globulin induction | 0.83 | (0.80 to 0.85) |
| iBox in African American population** | 0.80 | (0.74 to 0.85) |

| | | |
|---|---|---|
| iBox in non-African American population** | 0.84 | (0.80 to 0.89) |
| iBox adding recipient blood pressure profile post-transplant †† | 0.80 | (0.78 to 0.82) |
| iBox adding CNI blood through level at time of evaluation | 0.81 | (0.78 to 0.83) |

\* Histological diagnoses defined by the last update of the Banff international classification: antibody-mediated rejection, T-cell mediated rejection, BK virus nephropathy, primary nephropathy recurrence

†Highly sensitised patients defined by a panel of reactive antibodies >90%

‡ Transplant baseline characteristics are donor's age, donor's gender, donor's hypertension, donor's diabetes, recipient's age, recipient's gender, HLA mismatches, retransplantation and anti-HLA DSA at the time of transplantation.

\*\* African American recipient status was retrieved in the US participating centres databases (no data ethnicity allowed in the French development cohort database according to the French law & regulation). African Americans within the US validation cohort represented a total of 390 patients (27.31%)

Non-African Americans within the US validation cohort represented a total of 1,038 patients (72.69%)

†† blood profile is defined by systolic blood pressure measured at the time of risk assessment in log scale

**Table 4: Details of the Clinical trials depicting the population characteristics, clinical scenarios and interventions**

| STUDY | Trial #ID | Design | Clinical scenario | Target population | (n) | Time post-transplant of iBox risk score evaluation | Follow-up time post-transplant | iBox risk score C-Stat |
|---|---|---|---|---|---|---|---|---|
| CERTITEM* | **NCT 01079143** | Prospective, Randomised, open-label, multicentre trial | ISD minimisation | Recipients of renal transplants from a living or deceased donor | 194 | Median: 0.94 years IQR (0.92-0.98) | Median: 6.62 years IQR (2.82-7.34) | 0.88 |
| RITUX ERAH† | **Eudra CT 2007-003213-13** | Prospective, Randomised, multicentre, double-blind, placebo-controlled trial | Treatment of ABMR (preexisting DSA) | Recipients of renal transplants from a living or deceased donor with diagnosis of acute ABMR. | 38 | Median: 0.74 years IQR (0.53-1.10) | Median: 6.63 years IQR (4.03-7.69) | 0.77 |
| BORTEJECT‡ | **NCT 01873157** | Prospective, Randomised, placebo-controlled, double-blind, single-centre trial | Treatment of ABMR (*de novo* DSA) | Recipients of renal transplants from a living or deceased donor with post-transplant *de novo* DSA detection | 44 | Median: 6.61 years IQR (4.04-15.41) | Median: 7.75 years IQR (5.32-16.41) | 0.94 |

*: Rostaing, L., et al. "Fibrosis progression according to epithelial-mesenchymal transition profile: a randomised trial of everolimus versus CsA." American Journal of Transplantation 15.5 (2015): 1303-1312; †: Sautenet, B., et al. "One-year results of the effects of rituximab on acute antibody-mediated rejection in renal transplantation: RITUX ERAH, a multicentre double-blind randomised placebo-controlled trial." Transplantation 100.2 (2016): 391-399; ‡: Eskandary, Farsad, et al. "A Randomised Trial of Bortezomib in Late Antibody-Mediated Kidney Transplant Rejection." Journal of the American Society of Nephrology (2017): ASN-2017070818.

**Table 4: General transplant procedures and policies and allocation systems in the participating centres**

| Transplant Referral Centres | Allocation system | Deceased / living donor rate | Expanded criteria donor rate | Dual kidney transplantation program | Paired donor exchange national program | ABO incompatible program | HLA incompatible program | Standard induction therapy Protocols ATG: Anti-thymocyte Globulin IL2R: interleukin 2 receptor |
|---|---|---|---|---|---|---|---|---|
| **Paris Transplant Group Saint Louis, Necker, and Foch Hospitals, France** | ABM: *Agence Française Biomédecine**  | 84% / 16% | 42% | **YES** | **NO** | **YES** | **YES** | **Induction rate 100%** (ATG or anti-IL2R) |
| **Toulouse Hospital, France** | ABM: *Agence Française Biomédecine** | 88% / 12% | 41% | **NO** | **NO** | **YES** | **YES** | **Induction rate 85%** (ATG or anti-IL2R) |
| **Nantes Hospital, France** | ABM: *Agence Française Biomédecine** | 90% / 10% | 50% | **NO** | **NO** | **NO** | **NO** | **Induction rate 80%** (ATG or anti-IL2R) |
| **Lyon Hospital, France** | ABM: *Agence Française Biomédecine** | 93% / 7% | 24% | **YES** | **NO** | **YES** | **NO** | **Induction rate 100%** (ATG or anti-IL2R) |
| **Leuven Hospital, Belgium** | EuroTransplant: EU allocation system† | 94% / 6% | 30% | **NO** | **NO** | **YES** | **NO** | **Induction rate 40%** (anti-IL2R) |
| **Johns Hopkins Medical Institute, Baltimore, USA** | UNOS United Nations for Organ Sharing‡ | 49% / 51% | 13% | **NO** | **YES** | **YES** | **YES** | **Induction rate 100%** (ATG or anti-IL2R) |
| **Virginia, USA** | UNOS United Nations for Organ Sharing‡ | 27% / 73% | 10% | **NO** | **YES** | **YES** | **NO** | **Induction rate 100%** (ATG or anti-IL2R) |
| **Mayo Clinic, Rochester, USA** | UNOS United Nations for Organ Sharing‡ | 22% / 78% | 4% | **NO** | **YES** | **YES** | **YES** | **Induction rate 100%** (ATG or anti-IL2R) |

*http://sipg.sante.fr/portail/, †http://www.eurotransplant.org/, ‡http://www.unos.org/

EP 3 712 898 A1

**Table 6: Baseline characteristics of the European validation centres**

| | | Nantes (France) (n=632) | | Lyon (France) (n=608) | | Leuven (Belgium) (n=889) |
|---|---|---|---|---|---|---|
| | **n** | | **n** | | **n** | |
| **Recipient characteristics** | | | | | | |
| **Age** (years), mean (SD) | 632 | 50.38 (13.57) | 608 | 46.63 (13.28) | 889 | 53.42 (13.30) |
| **Gender male,** No. (%) | 632 | 404 (63.92) | 608 | 386 (63.49) | 889 | 543 (61.08) |
| **ESRD causes** | 632 | | 608 | | 889 | |
| Glomerulonephritis, No. (%) | | 179 (28.32) | | 151 (24.84) | | 254 (28.57) |
| Diabetes, No. (%) | | 55 (8.70) | | 188 (30.92) | | 73 (8.21) |
| Vascular, No. (%) | | 53 (8.39) | | 49 (8.06) | | 37 (4.16) |
| Other, No. (%) | | 345 (54.59) | | 220 (36.18) | | 525 (59.06) |
| **Donor characteristics** | | | | | | |
| **Age** (years), mean (SD) | 632 | 53.07 (14.99) | 603 | 44.08 (16.55) | 887 | 47.63 (14.89) |
| **Male gender,** No. (%) | 631 | 354 (56.10) | 605 | 395 (65.29) | 888 | 476 (53.60) |
| **Hypertension,** No. (%) | 620 | 185 (29.84) | 607 | 101 (16.64) | 649 | 164 (25.27) |
| **Diabetes mellitus,** No. (%) | 481 | 36 (7.48) | 343 | 11 (3.21) | 889 | 0 |
| **Creatinine > 1.5 mg/dL,** No. (%) | 631 | 80 (12.68) | 605 | 95 (15.70) | 700 | 18 (2.57) |
| **Donor type Deceased donor,** No. (%) | 632 | 576 (91.14) | 608 | 564 (92.76) | 889 | 834 (93.81) |
| **Death from cerebrovascular disease,** No. (%) | 576 | 323 (56.08) | 564 | 257 (45.57) | 834 | 413 (49.52) |
| **Expanded criteria donor,** No. (%) | 574 | 248 (43.21) | 608 | 142 (23.36) | 828 | 238 (28.74) |
| **Transplant baseline characteristics** | | | | | | |
| **Prior kidney transplant,** No. (%) | 632 | 101 (15.98) | 608 | 94 (15.46) | 889 | 127 (14.29) |
| **Cold ischemia time** (hours), mean (SD) | 632 | 18.75 (9.39) | 599 | 13.68 (5.85) | 862 | 14.37 (5.44) |
| **Delayed graft function*,** No. (%) | 630 | 213 (33.81) | 608 | 102 (16.78) | 889 | 161 (18.11) |
| **HLA-A/B/DR mismatch,** mean (SD), number | 632 | 3.28 (1.36) | 608 | 3.58 (1.35) | 843 | 2.75 (1.34) |

Abbreviations: ESRD: end-stage renal disease; HLA: human leucocyte antigen.
* Delayed graft function was defined as the use of dialysis in the first postoperative week

**Table 7: Baseline characteristics of the North-American validation centres**

| | | Johns Hopkins (USA) (n=580) | | Mayo Clinic (USA) (n=556) | | Virginia (USA) (n=292) |
|---|---|---|---|---|---|---|
| | **n** | | **n** | | **n** | |
| **Recipient characteristics** | | | | | | |
| **Age** (years), mean (SD) | 580 | 51.01 (14.70) | 556 | 52.19 (13.74) | 284 | 45.74 (12.88) |
| **Gender male,** No. (%) | 580 | 321 (55.34) | 556 | 340 (61.15) | 292 | 169 (57.88) |
| **ESRD causes** | 580 | | 556 | | 292 | |

(continued)

| | Johns Hopkins (USA) (n=580) | | Mayo Clinic (USA) (n=556) | | Virginia (USA) (n=292) | |
|---|---|---|---|---|---|---|
| | **n** | | **n** | | **n** | |
| **Recipient characteristics** | | | | | | |
| **Glomerulonephritis**, No. (%) | | 147 (25.34) | | 162 (29.14) | | 56 (19.18) |
| **Diabetes**, No. (%) | | 116 (20.00) | | 106 (19.06) | | 49 (16.78) |
| **Vascular**, No. (%) | | 97 (16.72) | | 63 (11.33) | | 89 (30.48) |
| **Other**, No. (%) | | 220 (37.93) | | 225 (40.47) | | 98 (33.56) |
| **Donor characteristics** | | | | | | |
| **Age** (years), mean (SD) | 580 | 40.11 (14.78) | 556 | 43.29 (13.00) | 284 | 38.39 (17.13) |
| **Male gender**, No. (%) | 580 | 279 (48.10) | 556 | 258 (46.40) | 284 | 157 (55.28) |
| **Hypertension**, No. (%) | 578 | 73 (12.63) | 429 | 50 (11.66) | 280 | 66 (23.57) |
| **Diabetes mellitus**, No. (%) | 577 | 30 (5.20) | 419 | 3 (0.7) | 280 | 14 (5.00) |
| **Creatinine > 1.5 mg/dL**, No. (%) | 281 | 79 (28.11) | 510 | 148 (29.02) | 284 | 57 (20.07) |
| **Donor type Deceased donor**, No. (%) | 580 | 283 (48.79) | 556 | 123 (22.12) | 292 | 214 (73.29) |
| **Death from cerebrovascular disease**, No. (%) | 283 | 88 (31.10) | 123 | 36 (29.27) | 212 | 70 (33.02) |
| **Expanded criteria donor**, No. (%) | 580 | 38 (6.55) | 556 | 5 (0.90) | 289 | 29 (10.03) |
| **Transplant baseline characteristics** | | | | | | |
| **Prior kidney transplant**, No. (%) | 580 | 99 (17.07) | 544 | 78 (14.34) | 284 | 58 (20.42) |
| **Cold ischemia time** (hours), mean (SD) | 541 | 10.54 (13.35) | 397 | 4.02 (6.97) | 274 | 15.44 (10.70) |
| **Delayed graft function***, No. (%) | 576 | 35 (6.08) | 556 | 3 (0.54) | 292 | 120 (41.10) |
| **HLA-A/B/DR mismatch**, mean (SD), number | 579 | 3.64 (1.73) | 556 | 3.18 (1.86) | 292 | 4.03 (1.61) |

Abbreviations: ESRD: end-stage renal disease; HLA: human leucocyte antigen.

* Delayed graft function was defined as the use of dialysis in the first postoperative week

**Table 8: Independent determinants of kidney allograft loss in the derivation cohort stratified by centre: multivariable analysis**

| | | Number of patients | Number of events | HR | 95% CI | p |
|---|---|---|---|---|---|---|
| **Time from transplant to evaluation (year)** | | 3,941 | 538 | 1.074 | (1.017-1.134) | **0.0108** |
| **eGFR (mL/min/1.73 m²)** | | 3,941 | 538 | 0.955 | (0.949-0.961) | **<0.0001** |
| **Proteinuria (log)** | | 3,941 | 538 | 1.527 | (1.414-1.648) | **<0.0001** |
| **Interstitial fibrosis/ Tubular atrophy (IFTA)** | 0/1 | 3,074 | 330 | 1 | | |
| | 2 | 550 | 115 | 1.287 | (1.029-1.610) | |
| | 3 | 317 | 93 | 1.712 | (1.321-2.220) | **0.0002** |

(continued)

| | | Number of patients | Number of events | HR | 95% CI | p |
|---|---|---|---|---|---|---|
| **Microcirculation Inflammation (g+ptc)** | **0-2** | 3,568 | 414 | 1 | | |
| | **3-4** | 299 | 90 | 1.484 | (1.142-1.930) | |
| | **5-6** | 74 | 34 | 2.017 | (1.358-2.997) | **0.0003** |
| **Interstitial inflammation and tubulitis (i+t)** | **0-2** | 3,559 | 447 | 1 | | |
| | **≥3** | 382 | 91 | 1.352 | (1.071-1.706) | **0.0111** |
| **Transplant Glomerulopathy (cg)** | **0** | 3,684 | 445 | 1 | | |
| | **≥1** | 257 | 93 | 1.480 | (1.140-1.921) | **0.0032** |
| **Anti-HLA donor-specific antibody mean** | **<500** | 3,265 | 387 | 1 | | |
| | **≥500 - 3,000** | 477 | 80 | 1.280 | (0.986-1.661) | |
| **fluorescence intensity** | **≥3,000 - 6,000** | 80 | 23 | 1.809 | (1.167-2.803) | |
| | **≥6,000** | 119 | 48 | 2.228 | (1.591-3.120) | **<0.0001** |

## Table 9: iBox risk score comparison of previously published risk scores

| STUDY | Trial Registration /protocol | Study Design | Population | Number of | External Validation | Time of risk evaluation | Follow-up time post- | Validation in therapeutic randomised controlled | Candidate Predictors | Data set qualification | Allograft phenotypes at the time | CSTAT validation in | Individual risk |
|---|---|---|---|---|---|---|---|---|---|---|---|---|---|
| Gonzales et al[*] | None | Retrospective | n=556 (1999 – 2008) | 1 | No | Fixed at 1 year after transplant | Median: not applicable | No | 17 | Not audited | Yes (Banff international classification) | 0.69[§] | No |
| Premaud et al[†] | None | Retrospective | n=664 (1984 – 2011) | 3 | Yes n=896 France only | Fixed at 1 year after transplant +2 adjustable variables | Median: 6.4 years | No | 12 | Not audited | No | 0.71[∥] | No |
| iBox Risk score trial | Clinical trial.gov #NCT03474003 | Prospective observational | n=4,000 (2000 – 2014) | 10 | Yes n=3,557 Europe and US | Time adjusted [‡] | Median: 7.65 years (IQR: 5.20-10.30) | Yes 3 RCT (NCT01079143, EudrCT 2007-003213-13 and NCT01873157) | 33 | Annual audit | Yes (Banff international classification) | - | iBox risk prediction score individual calculation tools for clinicians and patients |

[*] Gonzales MM, Bentall A, Kremers WK, Stegall MD, Borrows R. Predicting Individual Renal Allograft Outcomes Using Risk Models with 1-Year Surveillance Biopsy and Alloantibody Data. J Am Soc Nephrol. 2016;27(10):3165-74, [†] Premaud A, Filloux M, Gatault P, Thierry A, Buchler M, Munteanu E, et al. An adjustable predictive score of graft survival in kidney transplant patients and the levels of risk linked to de novo donor-specific anti-HLA antibodies. PloS one. 2017;12(7):e0180236, [‡] see **Figure 3** for the distribution of iBox time post-transplant risk evaluation, **Table 2B** for the inclusion of "time of risk evaluation post-transplant" in the final model, and **Figure 5** showing examples of time updated iBox risk evaluation in patients (Patient **#3** and **#4**)

## Table 10: Independent determinants of kidney allograft loss in the derivation cohort using histological diagnoses: multivariable analysis

| | Number of patients | Number of events | HR | 95% CI | p |
|---|---|---|---|---|---|
| **Time from transplant to evaluation (year)** | 3,997 | 548 | 1.097 | (1.043-1.153) | **0.0003** |
| **eGFR (mL/min/1.73 m²)** | 3,997 | 548 | 0.955 | (0.949-0.961) | **<0.0001** |

(continued)

|  | | Number of patients | Number of events | HR | 95% CI | p |
|---|---|---|---|---|---|---|
| Proteinuria (log) | | 3,997 | 548 | 1.552 | (1.443-1.670) | <0.0001 |
| Antibody-mediated rejection | No | 3,398 | 368 | 1 | - | |
| | Yes | 599 | 180 | 1.811 | (1.475-2.223) | <0.0001 |
| T-cell mediated rejection | No | 3,810 | 502 | 1 | - | |
| | Yes | 187 | 46 | 1.369 | (1.007-1.861) | 0.0453 |
| Nephropathy Recurrence | No | 3,867 | 510 | 1 | - | |
| | Yes | 130 | 38 | 1.680 | (1.199-2.355) | 0.0026 |
| BK virus associated nephropathy | No | 3,900 | 517 | 1 | | |
| | Yes | 97 | 31 | 1.450 | (1.000-2.107) | 0.0500 |
| Anti-HLA donor-specific antibody mean fluorescence intensity | <500 | 3,309 | 393 | 1 | - | |
| | ≥500 - 3,000 | 483 | 82 | 1.220 | (0.946-1.572) | |
| | ≥3,000 - 6,000 | 82 | 24 | 1.527 | (0.993-2.348) | |
| | ≥6,000 | 123 | 49 | 1.985 | (1.432-2.753) | 0.0003 |

## REFERENCES:

Throughout this application, various references describe the state of the art to which this invention pertains. The disclosures of these references are hereby incorporated by reference into the present disclosure.

1. Mills KT, Xu Y, Zhang W, Bundy JD, Chen CS, Kelly TN, et al. A systematic analysis of worldwide population-based data on the global burden of chronic kidney disease in 2010. Kidney Int. 2015;88(5):950-7.

2. Hill NR, Fatoba ST, Oke JL, Hirst JA, O'Callaghan CA, Lasserson DS, et al. Global Prevalence of Chronic Kidney Disease - A Systematic Review and Meta-Analysis. PLoS One. 2016;11(7):e0158765.

3. Evans RW, Manninen DL, Garrison LP, Jr., Hart LG, Blagg CR, Gutman RA, et al. The quality of life of patients with end-stage renal disease. The New England journal of medicine. 1985;312(9):553-9.

4. Meier-Kriesche HU, Schold JD, Srinivas TR, Kaplan B. Lack of improvement in renal allograft survival despite a marked decrease in acute rejection rates over the most recent era. American journal of transplantation : official journal of the American Society of Transplantation and the American Society of Transplant Surgeons. 2004;4(3):378-83.

5. Coemans M, Susal C, Dohler B, Anglicheau D, Giral M, Bestard O, et al. Analyses of the short- and long-term graft survival after kidney transplantation in Europe between 1986 and 2015. Kidney Int. 2018.

6. Perl J. Kidney transplant failure: failing kidneys, failing care? Clin J Am Soc Nephrol. 2014;9(7):1153-5.

7. Stegall MD, Morris RE, Alloway RR, Mannon RB. Developing New Immunosuppression for the Next Generation of Transplant Recipients: The Path Forward. American journal of transplantation : official journal of the American Society of Transplantation and the American Society of Transplant Surgeons. 2016;16(4):1094-101.

8. Vincenti F, Rostaing L, Grinyo J, Rice K, Steinberg S, Gaite L, et al. Belatacept and Long-Term Outcomes in Kidney Transplantation. The New England journal of medicine. 2016;374(4):333-43.

9. Kaplan B, Schold J, Meier-Kriesche HU. Poor predictive value of serum creatinine for renal allograft loss. American journal of transplantation : official journal of the American Society of Transplantation and the American Society of Transplant Surgeons. 2003;3(12):1560-5.

10. He X, Moore J, Shabir S, Little MA, Cockwell P, Ball S, et al. Comparison of the predictive performance of eGFR formulae for mortality and graft failure in renal transplant recipients. Transplantation. 2009;87(3):384-92.

11. Naesens M, Lerut E, Emonds MP, Herelixka A, Evenepoel P, Claes K, et al. Proteinuria as a Noninvasive Marker for Renal Allograft Histology and Failure: An Observational Cohort Study. J Am Soc Nephrol. 2016;27(1):281-92.

12. Yilmaz S, Tomlanovich S, Mathew T, Taskinen E, Paavonen T, Navarro M, et al. Protocol core needle biopsy and histologic Chronic Allograft Damage Index (CADI) as surrogate end point for long-term graft survival in multicenter studies. J Am Soc Nephrol. 2003;14(3):773-9.

13. Lefaucheur C, Loupy A, Hill GS, Andrade J, Nochy D, Antoine C, et al. Preexisting donor-specific HLA antibodies predict outcome in kidney transplantation. J Am Soc Nephrol. 2010;21(8):1398-406.

14. Moore J, He X, Shabir S, Hanvesakul R, Benavente D, Cockwell P, et al. Development and evaluation of a composite risk score to predict kidney transplant failure. Am J Kidney Dis. 2011;57(5):744-51.

15. Shabir S, Halimi JM, Cherukuri A, Ball S, Ferro C, Lipkin G, et al. Predicting 5-year risk of kidney transplant failure: a prediction instrument using data available at 1 year posttransplantation. Am J Kidney Dis. 2014;63(4):643-51.

16. Gonzales MM, Bentall A, Kremers WK, Stegall MD, Borrows R. Predicting Individual Renal Allograft Outcomes Using Risk Models with 1-Year Surveillance Biopsy and Alloantibody Data. J Am Soc Nephrol. 2016;27(10):3165-74.

17. Schold JD, Kaplan B. The elephant in the room: failings of current clinical endpoints in kidney transplantation. American journal of transplantation : official journal of the American Society of Transplantation and the American Society of Transplant Surgeons. 2010;10(5):1163-6.

18. https://www.eurotransplant.org

19. https://unos.org/

20. Eskandary F, Regele H, Baumann L, Bond G, Kozakowski N, Wahrmann M, et al. A Randomized Trial of Bortezomib in Late Antibody-Mediated Kidney Transplant Rejection. J Am Soc Nephrol. 2018;29(2):591-605.

21. Sautenet B, Blancho G, Buchler M, Morelon E, Toupance O, Barrou B, et al. One-year Results of the Effects of Rituximab on Acute Antibody-Mediated Rejection in Renal Transplantation: RITUX ERAH, a Multicenter Double-blind Randomized Placebo-controlled Trial. Transplantation. 2016;100(2):391-9.

22. Rostaing L, Hertig A, Albano L, Anglicheau D, Durrbach A, Vuiblet V, et al. Fibrosis progression according to epithelial-mesenchymal transition profile: a randomized trial of everolimus versus CsA. American journal of transplantation : official journal of the American Society of Transplantation and the American Society of Transplant Surgeons. 2015;15(5):1303-12.

23. Collins GS, Reitsma JB, Altman DG, Moons KG. Transparent Reporting of a multivariable prediction model for Individual Prognosis or Diagnosis (TRIPOD): the TRIPOD statement. Ann Intern Med. 2015;162(1):55-63.

24. Lamb KE, Lodhi S, Meier-Kriesche HU. Long-term renal allograft survival in the United States: a critical reappraisal. American journal of transplantation : official journal of the American Society of Transplantation and the American Society of Transplant Surgeons. 2011;11(3):450-62.

25. Fine JP, Gray RJ. A proportional hazards model for the subdistribution of a competing Risk. J Am Stat Assoc 1999;94:496-509.

26. Efron B. Bootstrap Methods: Another Look at the Jackknife. Ann. Stats. 1979;7(1):1-26.

27. Harrell FE, Jr., Lee KL, Mark DB. Multivariable prognostic models: issues in developing models, evaluating assumptions and adequacy, and measuring and reducing errors. Stat Med. 1996;15(4):361-87.

28. Pencina MJ, D'Agostino RB, Sr., Steyerberg EW. Extensions of net reclassification improvement calculations to measure usefulness of new biomarkers. Stat Med. 2011;30(1):11-21.

29. Uno H, Tian L, Cai T, Kohane IS, Wei LJ. A unified inference procedure for a class of measures to assess improvement in risk prediction systems with survival data. Stat Med. 2013;32(14):2430-42.

30. Cox DR. Note on Grouping. J Am Stat Assoc. 1957 ;52: 543-547.

31. Rao PS, Schaubel DE, Guidinger MK, Andreoni KA, Wolfe RA, Merion RM, et al. A comprehensive risk quantification score for deceased donor kidneys: the kidney donor risk index. Transplantation. 2009;88(2):231-6.

32. Hosmer DW LS. Applied Logistic Regression. 2nd ed: New York, NY: John Wiley & Sons; 2000.

33. Prasad V, Kim C, Burotto M, Vandross A. The Strength of Association Between Surrogate End Points and Survival in Oncology: A Systematic Review of Trial-Level Meta-analyses. JAMA Intern Med. 2015;175(8):1389-98.

34. Sellares J, de Freitas DG, Mengel M, Reeve J, Einecke G, Sis B, et al. Understanding the causes of kidney transplant failure: the dominant role of antibody-mediated rejection and nonadherence. American journal of transplantation : official journal of the American Society of Transplantation and the American Society of Transplant Surgeons. 2012;12(2):388-99.

35. Premaud A, Filloux M, Gatault P, Thierry A, Buchler M, Munteanu E, et al. An adjustable predictive score of graft survival in kidney transplant patients and the levels of risk linked to de novo donor-specific anti-HLA antibodies. PLoS One. 2017;12(7):e0180236.

**Claims**

1. A method of predicting whether a kidney transplant recipient is at risk of having allograft loss comprising the steps of a) assessing for said recipient a plurality of parameters; b) implementing an algorithm on data comprising the parameters assessed at step a) as to obtain an algorithm output; c) determining the probability of allograft loss from the algorithm output of step b) wherein said parameters are i) time of posttransplant risk evaluation; ii) allograft functional parameters that include estimated glomerular filtration rate and proteinuria; iii) allograft histological parameters that include interstitial fibrosis and tubular atrophy, glomerulitis and peritubular capillaritis, interstitial inflammation and tubulitis and transplant glomerulopathy; and iv) recipient immunological profile that includes the presence and level of the immunodominant circulating anti-HLA donor-specific antibodies.

2. The method of claim 1 wherein the algorithm is a machine learning algorithm.

3. The method of claim 1 wherein the output obtained by the algorithm at step b) is a score.

4. The method of claim 3 wherein the score classifies the patients into very high-risk, high-risk, intermediate-risk and low-risk group.

5. The method of claim 1 wherein the algorithm is performed by one or more programmable processors executing one or more computer programs to perform functions by operating on input data and generating output.

6. The method of claim 1 which comprises the step for selecting a therapeutic regimen or determining if a certain therapeutic regimen is more appropriate for a patient identified as having a high risk of allograft loss.

7. The method of claim 1 which comprises the steps of discriminating responder from non-responder with respect to a particular therapy.

8. The method of claim 1 wherein the transplant recipient is reenrolled in a clinical trial.

9. A method for monitoring the treatment of a transplant recipient comprising the steps of i) implementing the method of claim 1, and ii) if on a first (i.e. initial) testing the patient is identified as having a high risk of allograft rejection, the patient can be administered an appropriate therapeutic regiment, and iii) on a second testing (i.e. follow-up testing), the patient is identified as having low risk of allograft loss, the patient can be administered with a therapeutic regiment at a maintenance dose.

10. A method of monitoring patients enrolled in a clinical trial to provide a quantitative measure for the therapeutic efficacy of the therapy which is subject to the clinical trial by implementing the method of claim 1.

11. The method of claim 1 wherein the output of the algorithm (e.g. the score) constitutes a surrogate marker for use in the clinical trial for assessing the efficiency of a particular therapy.

* 59 missing patients (0.01%) due to at least one missing data point in the final model.

**Figure 1A**

**Figure 1B**

**Figure 1C**

Figures 2A-C

**Figures 2D-F**

**Figures 2G-I**

**Figure 3**

**Figure 4**

**Figure 5A**

**Figure 5B**

Figure 5C

Figure 5D

**Figure 6**

**Figure 7**

48

Figure 8

Europäisches
Patentamt

European
Patent Office

Office européen
des brevets

# EUROPEAN SEARCH REPORT

**Application Number**

EP 19 30 5348

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALEXANDRE LOUPY ET AL: "Molecular Microscope Strategy to Improve Risk Stratification in Early Antibody-Mediated Kidney Allograft Rejection", JOURNAL OF THE AMERICAN SOCIETY OF NEPHROLOGY., vol. 25, no. 10, 3 April 2014 (2014-04-03), pages 2267-2277, XP055618485, US ISSN: 1046-6673, DOI: 10.1681/ASN.2013111149 * page 2268, column 1 - page 2273, column 1 * | 1-11 | INV.<br>G16H20/40<br>G16H50/20<br>G16H50/30 |
| X | DENIS VIGLIETTI ET AL: "Value of Donor-Specific Anti-HLA Antibody Monitoring and Characterization for Risk Stratification of Kidney Allograft Loss", JOURNAL OF THE AMERICAN SOCIETY OF NEPHROLOGY., vol. 28, no. 2, 4 August 2016 (2016-08-04), pages 702-715, XP055390745, US ISSN: 1046-6673, DOI: 10.1681/ASN.2016030368 * page 703, column 1 - page 712, column 1; figures 1,4,5; tables 1-4 * | 1-11 | |
| X | WO 2019/014667 A1 (UNIV CALIFORNIA [US]) 17 January 2019 (2019-01-17) * paragraph [0011] - paragraph [0045] * * paragraph [0062] - paragraph [0095] * | 1-11 | TECHNICAL FIELDS SEARCHED (IPC)<br><br>G16H |

The present search report has been drawn up for all claims

| Place of search | Date of completion of the search | Examiner |
|---|---|---|
| Munich | 4 September 2019 | Menschner, Philipp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 30 5348

This annex lists the patent family members relating to the patent documents cited in the above-mentioned European search report.
The members are as contained in the European Patent Office EDP file on
The European Patent Office is in no way liable for these particulars which are merely given for the purpose of information.

04-09-2019

| Patent document cited in search report | Publication date | Patent family member(s) | Publication date |
|---|---|---|---|
| WO 2019014667 A1 | 17-01-2019 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7109304 B

**Non-patent literature cited in the description**

- **LEVEY A S ; BOSCH J P ; LEWIS J B ; GREENE T ; ROGERS N ; ROTH D.** A more accurate method to estimate glomerular filtration rate from serum creatinine: a new prediction equation. Modification of Diet in Renal Disease Study Group. *Ann. Intern. Med.,* 1999, vol. 130 (6), 461-70
- **LEONARD et al.** *Clinical Cancer Research,* vol. 10, 53Z7-5334
- **DE BENEDETTI et al.** *J Immunol,* 2001, vol. 166, 4334-4340
- **SUZUKI et al.** *Europ J of Immunol,* 1992, vol. 22 (8), 1989-1993
- **ROSTAING, L. et al.** Fibrosis progression according to epithelial-mesenchymal transition profile: a randomised trial of everolimus versus CsA. *American Journal of Transplantation,* 2015, vol. 15.5, 1303-1312
- **SAUTENET, B. et al.** One-year results of the effects of rituximab on acute antibody-mediated rejection in renal transplantation: RITUX ERAH, a multicentre double-blind randomised placebo-controlled trial. *Transplantation,* 2016, vol. 100.2, 391-399
- **ESKANDARY, FARSAD et al.** A Randomised Trial of Bortezomib in Late Antibody-Mediated Kidney Transplant Rejection. *Journal of the American Society of Nephrology,* 2017
- **MILLS KT ; XU Y ; ZHANG W ; BUNDY JD ; CHEN CS ; KELLY TN et al.** A systematic analysis of worldwide population-based data on the global burden of chronic kidney disease in 2010. *Kidney Int.,* 2015, vol. 88 (5), 950-7
- **HILL NR ; FATOBA ST ; OKE JL ; HIRST JA ; O'CALLAGHAN CA ; LASSERSON DS et al.** Global Prevalence of Chronic Kidney Disease - A Systematic Review and Meta-Analysis. *PLoS One,* 2016, vol. 11 (7), e0158765
- **EVANS RW ; MANNINEN DL ; GARRISON LP, JR. ; HART LG ; BLAGG CR ; GUTMAN RA et al.** The quality of life of patients with end-stage renal disease. *The New England journal of medicine,* 1985, vol. 312 (9), 553-9

- **MEIER-KRIESCHE HU ; SCHOLD JD ; SRINIVAS TR ; KAPLAN B.** Lack of improvement in renal allograft survival despite a marked decrease in acute rejection rates over the most recent era. *American journal of transplantation : official journal of the American Society of Transplantation and the American Society of Transplant Surgeons,* 2004, vol. 4 (3), 378-83
- **COEMANS M ; SUSAL C ; DOHLER B ; ANGLICHEAU D ; GIRAL M ; BESTARD O et al.** Analyses of the short- and long-term graft survival after kidney transplantation in Europe between 1986 and 2015. *Kidney Int.,* 2018
- **PERL J.** Kidney transplant failure: failing kidneys, failing care?. *Clin J Am Soc Nephrol.,* 2014, vol. 9 (7), 1153-5
- **STEGALL MD ; MORRIS RE ; ALLOWAY RR ; MANNON RB.** Developing New Immunosuppression for the Next Generation of Transplant Recipients: The Path Forward. *American journal of transplantation : official journal of the American Society of Transplantation and the American Society of Transplant Surgeons,* 2016, vol. 16 (4), 1094-101
- **VINCENTI F ; ROSTAING L ; GRINYO J ; RICE K ; STEINBERG S ; GAITE L et al.** Belatacept and Long-Term Outcomes in Kidney Transplantation. *The New England journal of medicine.,* 2016, vol. 374 (4), 333-43
- **KAPLAN B ; SCHOLD J ; MEIER-KRIESCHE HU.** Poor predictive value of serum creatinine for renal allograft loss. *American journal of transplantation : official journal of the American Society of Transplantation and the American Society of Transplant Surgeons,* 2003, vol. 3 (12), 1560-5
- **HE X ; MOORE J ; SHABIR S ; LITTLE MA ; COCKWELL P ; BALL S et al.** Comparison of the predictive performance of eGFR formulae for mortality and graft failure in renal transplant recipients. *Transplantation,* 2009, vol. 87 (3), 384-92
- **NAESENS M ; LERUT E ; EMONDS MP ; HERELIXKA A ; EVENEPOEL P ; CLAES K et al.** Proteinuria as a Noninvasive Marker for Renal Allograft Histology and Failure: An Observational Cohort Study. *J Am Soc Nephrol.,* 2016, vol. 27 (1), 281-92

- **YILMAZ S ; TOMLANOVICH S ; MATHEW T ; TASKINEN E ; PAAVONEN T ; NAVARRO M et al.** Protocol core needle biopsy and histologic Chronic Allograft Damage Index (CADI) as surrogate end point for long-term graft survival in multicenter studies. *J Am Soc Nephrol.,* 2003, vol. 14 (3), 773-9
- **LEFAUCHEUR C ; LOUPY A ; HILL GS ; ANDRADE J ; NOCHY D ; ANTOINE C et al.** Preexisting donor-specific HLA antibodies predict outcome in kidney transplantation. *J Am Soc Nephrol.,* 2010, vol. 21 (8), 1398-406
- **MOORE J ; HE X ; SHABIR S ; HANVESAKUL R ; BENAVENTE D ; COCKWELL P et al.** Development and evaluation of a composite risk score to predict kidney transplant failure. *Am J Kidney Dis.,* 2011, vol. 57 (5), 744-51
- **SHABIR S ; HALIMI JM ; CHERUKURI A ; BALL S ; FERRO C ; LIPKIN G et al.** Predicting 5-year risk of kidney transplant failure: a prediction instrument using data available at 1 year posttransplantation. *Am J Kidney Dis.,* 2014, vol. 63 (4), 643-51
- **GONZALES MM ; BENTALL A ; KREMERS WK ; STEGALL MD ; BORROWS R.** Predicting Individual Renal Allograft Outcomes Using Risk Models with 1-Year Surveillance Biopsy and Alloantibody Data. *J Am Soc Nephrol.,* 2016, vol. 27 (10), 3165-74
- **SCHOLD JD ; KAPLAN B.** The elephant in the room: failings of current clinical endpoints in kidney transplantation. *American journal of transplantation : official journal of the American Society of Transplantation and the American Society of Transplant Surgeons,* 2010, vol. 10 (5), 1163-6
- **ESKANDARY F ; REGELE H ; BAUMANN L ; BOND G ; KOZAKOWSKI N ; WAHRMANN M et al.** A Randomized Trial of Bortezomib in Late Antibody-Mediated Kidney Transplant Rejection. *J Am Soc Nephrol.,* 2018, vol. 29 (2), 591-605
- **SAUTENET B ; BLANCHO G ; BUCHLER M ; MORELON E ; TOUPANCE O ; BARROU B et al.** One-year Results of the Effects of Rituximab on Acute Antibody-Mediated Rejection in Renal Transplantation: RITUX ERAH, a Multicenter Double-blind Randomized Placebo-controlled Trial. *Transplantation,* 2016, vol. 100 (2), 391-9
- **ROSTAING L ; HERTIG A ; ALBANO L ; ANGLICHEAU D ; DURRBACH A ; VUIBLET V et al.** Fibrosis progression according to epithelial-mesenchymal transition profile: a randomized trial of everolimus versus CsA. *American journal of transplantation : official journal of the American Society of Transplantation and the American Society of Transplant Surgeons,* 2015, vol. 15 (5), 1303-12
- **COLLINS GS ; REITSMA JB ; ALTMAN DG ; MOONS KG.** Transparent Reporting of a multivariable prediction model for Individual Prognosis or Diagnosis (TRIPOD): the TRIPOD statement. *Ann Intern Med.,* 2015, vol. 162 (1), 55-63
- **LAMB KE ; LODHI S ; MEIER-KRIESCHE HU.** Long-term renal allograft survival in the United States: a critical reappraisal. *American journal of transplantation : official journal of the American Society of Transplantation and the American Society of Transplant Surgeons,* 2011, vol. 11 (3), 450-62
- **FINE JP ; GRAY RJ.** A proportional hazards model for the subdistribution of a competing Risk. *J Am Stat Assoc,* 1999, vol. 94, 496-509
- **EFRON B.** Bootstrap Methods: Another Look at the Jackknife. *Ann. Stats.,* 1979, vol. 7 (1), 1-26
- **HARRELL FE, JR. ; LEE KL ; MARK DB.** Multivariable prognostic models: issues in developing models, evaluating assumptions and adequacy, and measuring and reducing errors. *Stat Med.,* 1996, vol. 15 (4), 361-87
- **PENCINA MJ ; D'AGOSTINO RB, SR. ; STEYERBERG EW.** Extensions of net reclassification improvement calculations to measure usefulness of new biomarkers. *Stat Med.,* 2011, vol. 30 (1), 11-21
- **UNO H ; TIAN L ; CAI T ; KOHANE IS ; WEI LJ.** A unified inference procedure for a class of measures to assess improvement in risk prediction systems with survival data. *Stat Med.,* 2013, vol. 32 (14), 2430-42
- **COX DR.** Note on Grouping. *J Am Stat Assoc.,* 1957, vol. 52, 543-547
- **RAO PS ; SCHAUBEL DE ; GUIDINGER MK ; ANDREONI KA ; WOLFE RA ; MERION RM et al.** A comprehensive risk quantification score for deceased donor kidneys: the kidney donor risk index. *Transplantation,* 2009, vol. 88 (2), 231-6
- **HOSMER DW LS.** Applied Logistic Regression. John Wiley & Sons, 2000
- **PRASAD V ; KIM C ; BUROTTO M ; VANDROSS A.** The Strength of Association Between Surrogate End Points and Survival in Oncology: A Systematic Review of Trial-Level Meta-analyses. *JAMA Intern Med.,* 2015, vol. 175 (8), 1389-98
- **SELLARES J ; DE FREITAS DG ; MENGEL M ; REEVE J ; EINECKE G ; SIS B et al.** Understanding the causes of kidney transplant failure: the dominant role of antibody-mediated rejection and nonadherence. *American journal of transplantation : official journal of the American Society of Transplantation and the American Society of Transplant Surgeons,* 2012, vol. 12 (2), 388-99
- **PREMAUD A ; FILLOUX M ; GATAULT P ; THIERRY A ; BUCHLER M ; MUNTEANU E et al.** An adjustable predictive score of graft survival in kidney transplant patients and the levels of risk linked to de novo donor-specific anti-HLA antibodies. *PLoS One,* 2017, vol. 12 (7), e0180236